# EUROPEAN PATENT APPLICATION

(11) **EP 2 019 318 A1**
(43) Date of publication of application: **28.01.2009**
(21) Application number: 07113365.6
(22) Date of filing: 27.07.2007
(51) Int. Cl.: G01N 33/50, G01N 33/68

(54) **Protein markers for cardiovascular events**

(71) Applicant: Erasmus University Medical Center Rotterdam, 3015 GE Rotterdam (NL)
(72) Inventor: The designation of the inventor has not yet been filed
(74) Representative: van Loon, C.J.J.

(57) **Abstract**

The present invention relates to biomarkers in atherosclerotic plaque associated with an increased risk of suffering a cardiovascular event. The invention further relates to a method for predicting the risk of a subject suffering a cardiovascular event comprising detecting a biomarker according to the invention in (a sample of) atherosclerotic plaque from said subject. The invention further relates to diagnostic and pharmaceutical compositions and to methods of treating subjects having increased risk of suffering a cardiovascular event.

## Description

### FIELD OF THE INVENTION

The invention relates to the field of diagnostics, more particular to the prognosis of risks of cardiovascular events such as stroke, myocardial infarction (heart attack), cerebral bleeding and other major abnormalities occurring in the blood vessels. In particular, the present invention relates to the typing of plaques in the blood vessels, especially in the carotid arteries, and predicting the occurrence of restenosis.

### BACKGROUND TO THE INVENTION

The number one cause of mortality in Western Europe is atherosclerotic cardiovascular disease. This disease starts with the formation of atherosclerotic plaques in the blood vessels, and possibly evolves into cardiovascular events such as cerebrovascular stroke and cardiovascular obstructions resulting in myocardial infarctions. These continue to be a major burden to health care expenditure and treatment requires the most expensive and intensive forms of medical care available today. For a major part, this is due to diagnostic limitations. A pressing need therefore exists for adequate diagnostic tools to better resolve the molecular diversity of this disease and to stratify patients having a high risk for a cardiovascular event at an early time point.

The difference between patients that have suffered a major cardiovascular event and matched controls depends largely on genetic and epigenetic differences between individual patients. Molecular tests that can identify patients at risk for such events are limited and imaging is mainly focused on the classical histological concept of the vulnerable plaque, a concept that does not take the likelihood of an event into account. Within this perspective, plaque proteins are remarkably unexplored as markers or targets.

Few protein markers have been suggested as markers for predicting a higher risk to cardiovascular events. One of them is the C-reactive protein, which is said to be indicative for a higher risk of coronary events, stroke, peripheral vascular disease, and type 2 diabetes mellitus. Recently, also pentraxin 3 (PTX3) has been identified as a predictive marker protein.

However, there is still need for more and more reliable biomarkers for predicting the risk to major cardiovascular events.

### SUMMARY OF THE INVENTION

The present inventors have now discovered that the protein constitution in atherosclerotic plaque samples from subjects that have suffered a cardiovascular event prior to or following the moment of sampling, differs from that in patients who have not suffered such a cardiovascular event, and that this difference can be used for prognosis of patients. This surprising finding now provides a method for predicting the risk of a cardiovascular event in a patient, based on the detection in atherosclerotic plaque samples of proteins with prognostic value, herein after referred to as biomarkers or differentially present proteins.

In a first aspect, the present invention now provides a biomarker in atherosclerotic plaque associated with an increased risk of suffering a cardiovascular event. The biomarker of the invention may be used to type atherosclerotic plaque samples in methods of the present invention described in more detail below. By detecting the presence of this biomarker in plaque or in a sample of plaque from a subject, it is possible to predict if the subject has an increased risk of suffering a stroke or myocardial infarction.

The biomarkers of the present invention were discovered by large scale epidemiological studies involving typing of plaque samples and collecting information on the medical condition of the subjects donating the plaque, in particular the suffering of cardiovascular events following the sampling of the plaque during a period of 3 years. Predictive correlations could be established.

The biomarkers of the present invention are protein markers. It was found that the various proteins in plaque associated with an increased risk of suffering a cardiovascular event could be assigned to one of various specific metabolic pathways or molecular networks, on the basis of the role of these proteins in the network. Using dedicated software tools (Ingenuity® Pathways Analysis (IPA)), network functions could be assigned to processes as varied as cell-to-cell signalling and interaction, immune response, neurological disease, cellular movement and cancer, to protein degradation and reproductive system disease. Yet, clear networks could be identified to which these and other proteins belong. It is an aspect of the present invention that also other proteins from these networks are candidate biomarkers for use in aspects of the invention.

A biomarker of the present invention comprises at least one protein that is a member of at least one Ingenuity® network selected from the group consisting of:
a) the Ingenuity® network defined by the proteins ACTR2, CAPN2, CAST, FSCN1, HCLS1, HNRPA2B1, HNRPC, HSP90AA1, IGFBP7, MYLK, RAC1, S100A8, SERPINF1, SNCA, SPP1, SSB, STIP1, UCHL1, and YWHAQ;
b) the Ingenuity® network defined by the proteins AHNAK, AKR1A1, C1QB, C1QC, CBR3, CFD, CORO1C, FBP1, HNRPC, HNRPD, LAMP1, NEDD8, PSME2, SERPINC1, SPTA1, and TNC;
c) the Ingenuity® network defined by the proteins C19ORF10, CAPZA1 (includes EG:829), CAPZA2, CAPZB, DDX23, EEF1A2, FSCN1, GOLGA4, HSPA5, KRT1, M6PRBP1, NPC2, PGD, S100A8, SNIP1, and SPAN1;
d) the Ingenuity® network defined by the proteins ADH5, AK1, CALU (includes EG:813), CRYZ, DPYSL3, EIF3S3, HNRPA2B1, IGFALS, IGHG1, MRLC2, NP, PDLIM2, and TXNRD1;
e) the Ingenuity® network defined by the proteins AGC1, ANXA2, C6, CLTC, ERAF, FABP4, ITGB1, LMNA, RPS5, SPTB, THBS2, and TNC;
f) the Ingenuity® network defined by the proteins CLEC3B, HAPLN1, HNRPD, MDH2, PCOLCE, PSMA1, PSMD9, RCN1, SERPINC1, TBCA, TNC, and TRAP1;
g) the Ingenuity® network defined by protein RNPEP, and
h) the Ingenuity® network defined by the protein TWF2.

The above mentioned Ingenuity® networks are defined using Ingenuity Pathway Analysis 5.0 uploading the International Protein Index (IPI) identity of the proteins that were differentially expressed in atherosclerotic plaques between patients that had a cardiovascular event and age and sex matched patients that did not have an event (see for details example 1). Based on this list, the Ingenuity Pathways Analysis application leverages the Ingenuity Pathways Knowledge Base (IPKB) to provide insights into observed gene expression changes across biological samples. IPA dynamically computes a large "global" molecular network based on hundreds of thousands of curated direct and indirect physical and functional interactions. These data are stored as findings in the IPKB. Orthologous mammalian genes, proteins, protein complexes, groups and endogenous chemicals are eligible for network generation. The algorithms in IPA help select sub-parts of this global network (referred to as "networks") that are relevant to the experiment.

The following criteria play a role in generating the networks in the Ingenuity Pathways Analysis application:
1. The uploaded differentially expressed atherosclerotic plaque proteins between patients that had a cardiovascular event and age and sex matched patients that did not have an event on the "Create Analysis" page before running the analysis. Proteins that are designated in this group and which interact with other molecules in the IPKB are identified as Network Eligible Proteins. Network Eligible proteins serve as the "seeds", or focal points, for generating networks.
2. Networks are preferentially enriched for Network Eligible proteins with the most extensive interactions, and for which interactions are specific with the other genes/proteins in the network (rather than genes/proteins that interact with a broad selection of genes/proteins throughout the IPKB).
3. Additional proteins from the dataset are then added to the networks. Finally, proteins from the IPKB are added to the growing networks. In the current version of the application, there is a cutoff of 35 genes per network to keep networks to a usable size.
4. Networks are scored for the likelihood of finding the Network Eligible Genes in that given network. The higher the score, the lower the probability that you would find the Network Eligible Genes you see in a given network by random chance.

Additional pathway analysis revealed that the above-defined networks formed one super-network, consisting of 6 of the above-referred pathway networks. In a preferred embodiment of the present invention, said biomarker is a member the single Ingenuity® network composed of the networks as defined under a)-f).

In another preferred embodiment, said biomarker comprises at least one protein selected from the group consisting of ACTR2, ADH5, AGC1, AHNAK, AK1, AKR1A1, ANXA2, ANXA6, C19ORF10, C1QB, C1QC, C6, CALU (includes EG:813), CAPN2, CAPZA1 (includes EG:829), CAPZA2, CAPZB, CAST, CBR3, CDC37, CFD, CLEC3B, CLTC, CORO1C, CRYZ, DDAH2, DDX23, DPYSL3, EEF1A2, EFHD1, EIF3S3, ERAF, FABP4, FBP1, FSCN1, GOLGA4, HAPLN1, HCLS1, HNRPA2B1, HNRPC, HNRPD, HSP90AA1, HSPA5, IGFALS, IGFBP7, IGHG1, ITGB1, KRT1, LAMP1, LMNA, M6PRBP1, MDH2, MRLC2, MYLK, NEDD8, NP, NPC2, PCOLCE, PDLIM2, PGD, PSMA1, PSMD9, PSME2, RAC1, RCN1, RNPEP, RPS5, S100A8, SERPINA6, SERPINC1, SERPINF1, SNCA, SNIP1, SPP1, SPTA1, SPTAN1, SPTB, SSB, STIP1, TBCA, THBS2, TNC, TRAP1, TWF2, TXNRD1, UCHL1, and YWHAQ. More preferably, the biomarker comprises at least 2, 3, 4, or 5, still more preferably 6, 7, 8, 9, or 10, still more preferably 15, 20, 25, 30, 35, 40, 45, 50, 55, 60, 65, 75 or 80 of the proteins referred to above. Alternatively, the biomarker comprises all of the above proteins.

In another preferred embodiment, said biomarker comprises at least one protein selected from the group consisting of ACTR2, ADH5, AGC1, AHNAK, AK1, AKR1A1, ANXA2, ANXA6, C19ORF10, C1QB, C1QC, C6, CALU (includes EG:813), CAPN2, CAPZA1 (includes EG:829), CAPZA2, CAPZB, CAST, CBR3, CDC37, CFD, CLEC3B, CLTC, CORO1C, CRYZ, DDX23, DPYSL3, EEF1A2, EIF3S3, ERAF, FABP4, FBP1, FSCN1, GOLGA4, HAPLN1, HCLS1, HNRPA2B1, HNRPC, HNRPD, HSP90AA1, HSPA5, IGFALS, IGFBP7, IGHG1, ITGB1, KRT1, LAMP1, LMNA, M6PRBP1, MDH2, MRLC2, MYLK, NEDD8, NP, NPC2, PCOLCE, PDLIM2, PGD, PSMA1, PSMD9, PSME2, RAC1, RCN1, RPS5, S100A8, SERPINC1, SERPINF1, SNCA, SNIP1, SPP1, SPTA1, SPTAN1, SPTB, SSB, STIP1, TBCA, THBS2, TNC, TRAP1, TXNRD1, UCHL1, and YWHAQ. In another preferred embodiment, said biomarker comprises at least one protein selected from the group consisting of ADH5, AK1, ANXA2, ANXA6, FABP4, HNRPD, ITGB1, LMNA, RAC1, SERPINC1, SERPINF1, SPP1, SPTB, THBS2, TXNRD1, and UCHL1.

In a most preferred embodiment, said biomarker is SPP1 (osteopontin).

In another aspect, the present invention provides a method for predicting the risk of a subject developing a medical condition. Said method comprises the step of detecting a biomarker of the present invention as defined above in (a sample of) atherosclerotic plaque from said subject.

In a preferred embodiment of this method said atherosclerotic plaque is atherosclerotic plaque in a coronary, femoral and/or carotid artery.

In another preferred embodiment of this method, said medical condition is a cardiovascular event. Cardiovascular events comprise the following pathologies:
- Vascular death or Sudden death: (unexpected death occurring within 1 hour after onset of symptoms or within 24 hours given convincing circumstantial evidence) or death from stroke, myocardial infarction, congestive failure or rupture of abdominal aortic aneurysm.
- Non fatal stroke: Relevant clinical features which have caused an increase in handicap of at least one grade on the modified Ranking scale, accompanied by a fresh infarct or a hemorrhage on a repeat CT scan.
- Non fatal myocardial infarction: At least two of the following: (1) Chest pain for at least 20 min, not disappearing after administration of nitrates; (2) ST-elevation >1 mm in two following leads or a left bindle branch block on the ECG; (3) CK elevation of at least two times the normal value of CK and a MB fraction >5% of the total CK.
- Non fatal rupture of an abdominal aortic aneurysm
- Rupture of abdominal aortic aneurysm confirmed by laparatomy.
- Vascular intervention: Any vascular intervention to treat clinically relevant ischemic disease in coronary, carotid, iliacal, femoral and crural arteries that was not yet planned at inclusion.
- Restenosis (defined as >70% luminal narrowing of the operated carotid artery corresponding with a peak flow velocity >210 cm/sec.)

In another aspect, the present invention provides a method for predicting the risk of a test subject developing a medical condition comprising the steps of:
a) typing atherosclerotic plaque, comprising the steps of:
   - measuring the amount of at least one protein in a first (sample of) atherosclerotic plaque of a reference subject at risk of developing a medical condition and in a second (sample of) atherosclerotic plaque of a control subject not at risk of developing said medical condition, or providing a protein profile for said first and second (sample of) atherosclerotic plaque,
   - determining whether said at least one protein is differentially present in said first (sample of) atherosclerotic plaque compared to said second (sample of) atherosclerotic plaque, or determining the differential protein expression profile between said first and second (sample of) atherosclerotic plaque,
   - correlating the risk of developing said medical condition to said differentially present protein or to said differential protein expression profile,
   - identifying said differentially present protein or said differential protein expression profile as a biomarker in case said correlation is statistically significant,
   and
b) detecting said biomarker in (a sample of) atherosclerotic plaque from said test subject, wherein said test subject is at risk of developing said medical condition when said biomarker is present in said (sample of) atherosclerotic plaque. In a preferred embodiment of this method said medical condition is a cardiovascular event.

A control subject is usually a sex and age-matched control patient.

In any of the above methods of the invention, the step of measuring the amount of at least one protein; providing a protein profile, and/or detecting said biomarker, is performed by using analytical methods for showing the presence and/or concentration of a protein in a sample, e.g. mass spectrometry, protein chip array analysis, antibody array analysis, immunoassay analysis, MRI, NMR, ultrasound spectroscopy, Raman spectroscopy and/or infra red spectroscopy.

In a further aspect, the present invention provides diagnostic reagent capable of specifically binding to a biomarker of the present invention. Preferably said diagnostic reagent is an antibody or a derivative thereof.

In a further aspect, the present invention provides a diagnostic composition comprising a diagnostic reagent of the invention.

In yet a further aspect, the present invention provides a kit of parts for performing a method of the present invention, comprising at least one biomarker as defined herein above, or an antibody that binds specifically to said biomarker, said kit of parts optionally further comprising one or more of the following:
- at least one reference or control sample;
- information on the reference value for the biomarker;
- at least one peptide capable of binding to said antibody;
- at least one detectable marker for detecting binding between said biomarker and said antibody.

In another aspect, the present invention provides an antibody microarray for performing a method of the present invention, comprising antibodies that bind specifically to at least two biomarkers as defined herein above bound to a solid support.

In yet another aspect, the present invention provides the use of a biomarker as defined herein above for predicting the risk of a cardiovascular event in a subject.

In yet another aspect, the present invention provides a method of treating a subject having an increased risk of suffering a cardiovascular event, said method comprising using a biomarker as defined herein above as a therapeutic target. Preferably, said use of said biomarker as a therapeutic target comprises decreasing the amount of at least one protein that is over-expressed in subjects having an increased risk of suffering a cardiovascular event, or increasing the amount of at least one protein that is under-expressed in subjects having an increased risk of suffering a cardiovascular event. More preferably said protein that is over-expressed is SPP1 (osteopontin). In a further aspect, the present invention provides a pharmaceutical composition for the treatment of an increased risk of suffering a cardiovascular event, comprising at least one inhibitor compound selected from:
- an antibody or derivative thereof directed against the biomarker of any one of claims 1-6, preferably a biomarker expressed on the cell membrane, and said derivative preferably being selected from the group consisting of scFv fragments, Fab fragments, chimeric antibodies, bifunctional antibodies, intrabodies, and other antibody-derived molecules;
- a biomarker of any one of claims 1-6
- a small molecule interfering with the biological activity of said biomarker.
- an antisense molecule, in particular an antisense RNA or antisense oligodeoxynucleotide.
- an RNAi molecule and
- a ribozyme,
and a suitable excipient, carrier or diluent.

In a further aspect, the present invention provides a method of treating a subject, comprising administering to said subject the pharmaceutical composition of the invention in an amount effective to decrease or prevent the risk of said subject suffering a cardiovascular event.

### LEGENDS TO THE FIGURES

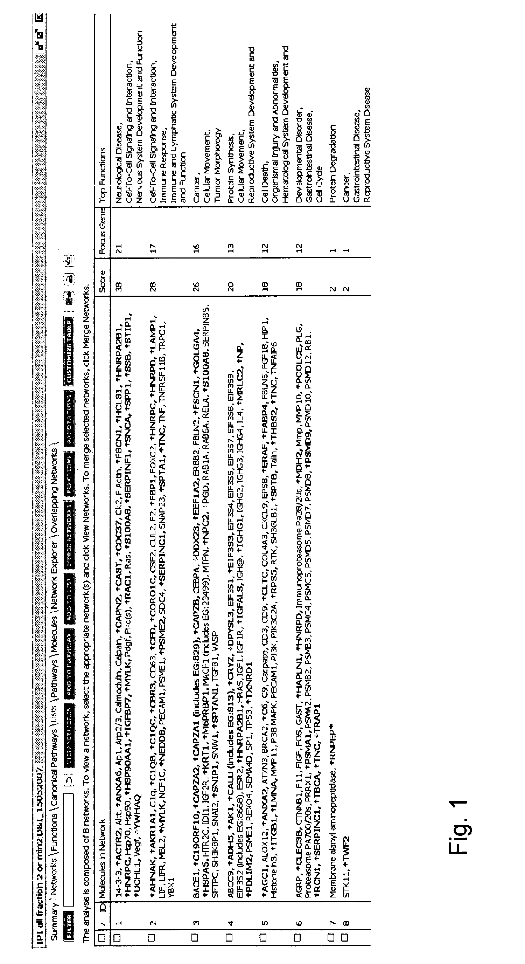

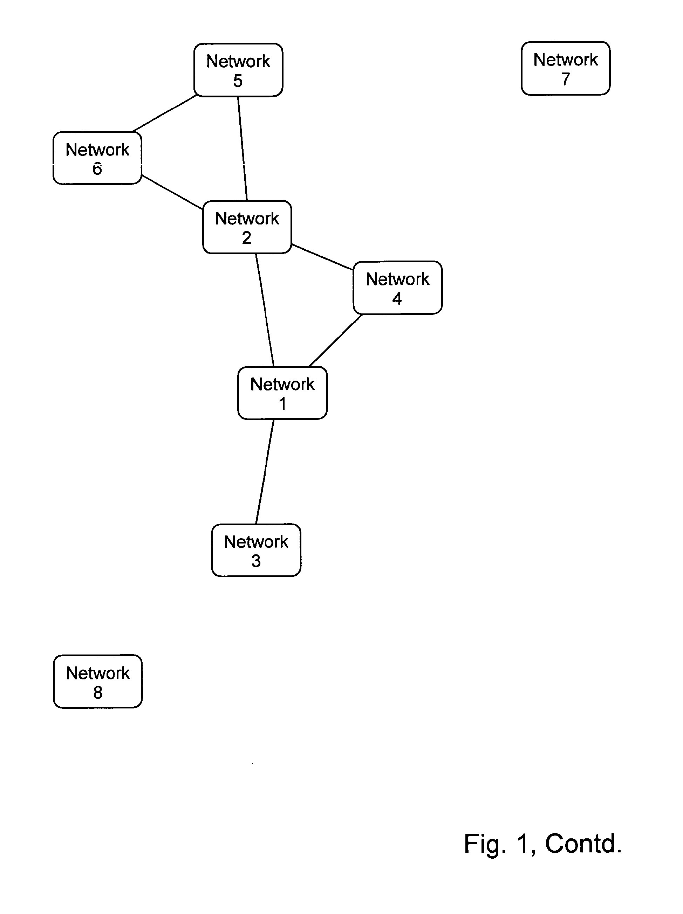

Fig. 1. Ingenuity™ analysis of human plaque proteins that were differentially expressed between patients that had a major cardiovascular events within 2 years after the removal of the carotid plaque (carotid atherectomy) and sex and age matched controls. Proteins were included into 6 networks that are connected to each other.

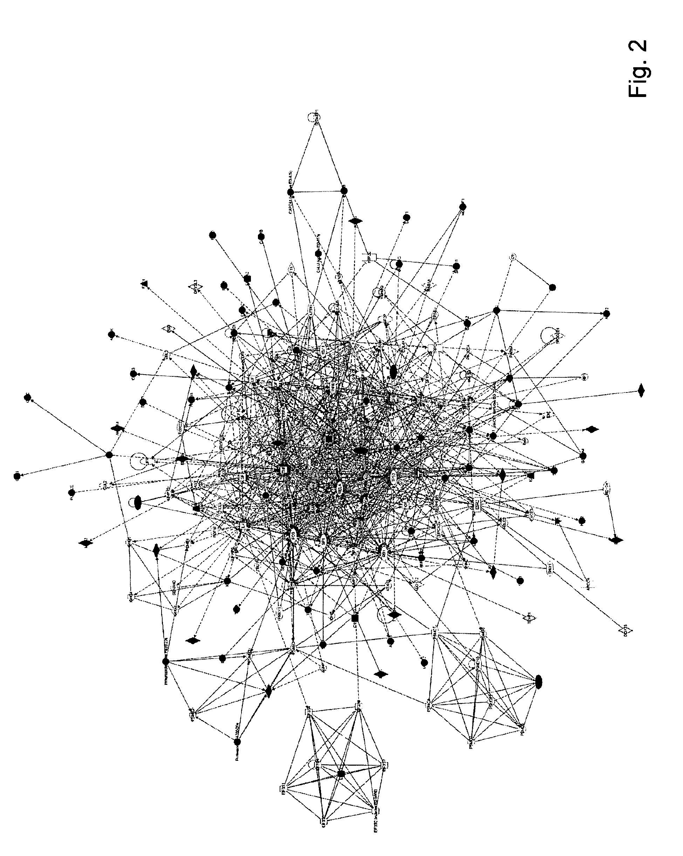

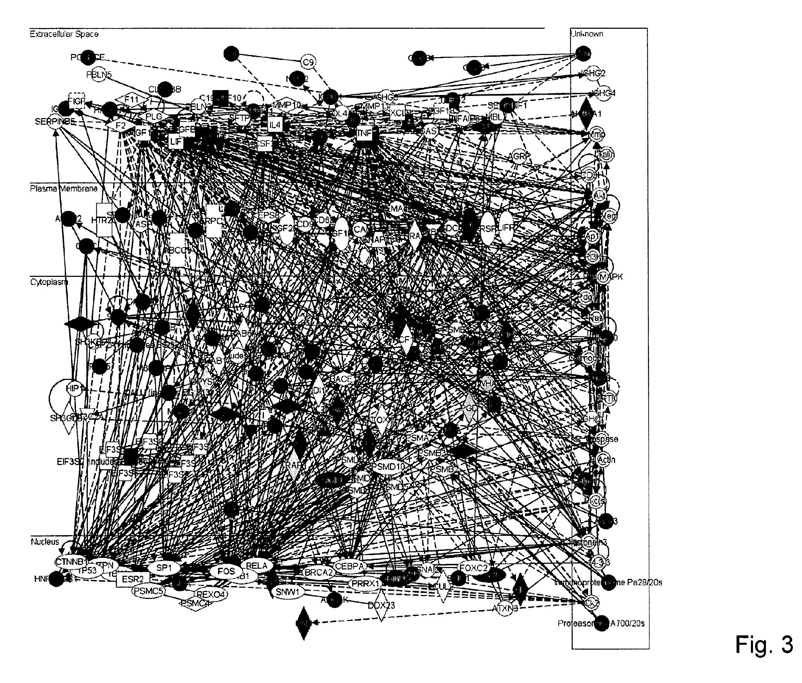

Fig. 2. Ingenuity™ network showing direct and indirect relationships between the 82 selected differential expressed proteins. Red is more (twice detected using the mass spectrometry analysis in the pooled event group but not in the matched control group in events), Green is more in controls (twice detected using the mass spectrometry analysis in the pooled control group but not in the matched event group. Fig. 3. Ingenuity™ network showing direct and indirect relationships between the 82 selected differential expressed proteins rearranged according to their location in the cell. Indications as in Fig. 2.

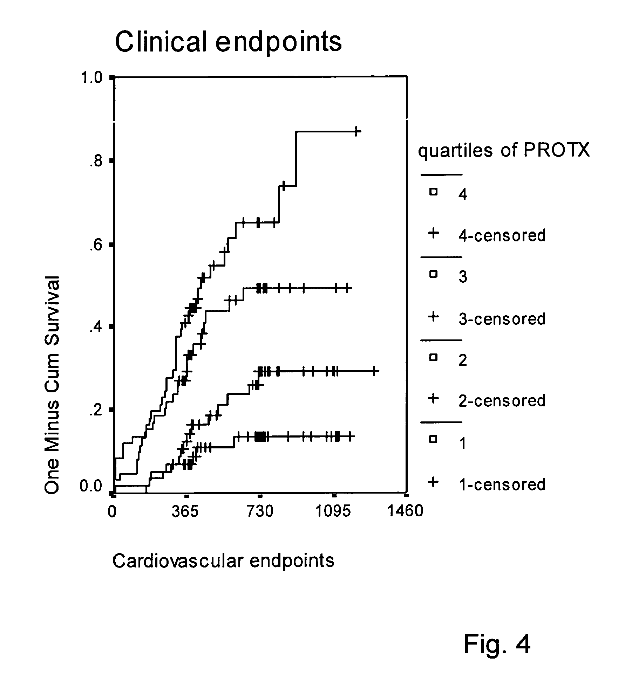

Fig. 4. Clinical cardiovascular events in time (days) of Atheroexpress patients related to quartiles of Osteopontin (OPN) levels. The green line (levelling off below .2) is the quartile 0-25% with the lowest OPN levels. The purple line (levelling off below .4) is the quartile 25-50%, the yellow line (levelling off below .6) is the quartile of 50-75% and the black line (levelling off above .8) is the quartile 75-100% of the highest OPN levels

### DETAILED DESCRIPTION OF THE INVENTION

### Terminology

The term "test subject at risk of developing a medical condition" as used herein, refers to a subject having a medical record wherein the incidence or frequency of the medical condition is statistically significantly higher than in the medical record of a control subject. The medical condition is preferably a cardiovasular event. The incidence or frequency of the medical condition is preferably determined in a time period following the step of providing the plaque sample from the subject. Said time period is preferably 1-10 years, more preferably 1-3 years.

The terms "polypeptide," "peptide" and "protein" are used interchangeably herein to refer to a polymer of amino acid residues. The terms apply to amino acid polymers in which one or more amino acid residue is an analog or mimetic of a corresponding naturally occurring amino acid, as well as to naturally occurring amino acid polymers. Polypeptides can be modified, e.g., by the addition of carbohydrate residues to form glycoproteins. The terms "polypeptide", "peptide" and "protein" include glycoproteins and proteins comprising any other modification, as well as non-glycoproteins and proteins that are otherwise unmodified.

"Protein profile", as used herein, refers to the collection of proteins, protein fragments, or peptides present in a sample. The protein profile may comprise the identities (e.g., specific names or amino acid sequence identities of known proteins, or molecular weights or other descriptive information about proteins that have not been further characterized) of the proteins in a collection, without reference to quantity present. In other embodiments, a protein profile includes quantitative information for the proteins represented in a sample.

"Quantitation", as used herein in reference to proteins in a profile refers to the determination of the amount of a particular protein or peptide present in a sample. Quantitation can be either in absolute amount (e.g., µg/ml) or a relative amount (e.g., relative intensity of signals).

"Marker" and "Biomarker" are used interchangeably to refer to a polypeptide that is differentially present in a samples taken from two different subjects, e.g., from a test subject or patient having (a risk of developing) a particular medical condition, such as cardiovascular events, compared to a comparable sample taken from a control subject (e.g., a subject not having (a risk of developing) a particular medical condition; a normal or healthy subject).

The phrase "differentially present" refers to differences in the quantity or frequency (incidence of occurrence) of a marker present in a sample taken from a test subject as compared to a control subject. For example, a marker can be a polypeptide that is present at an elevated level or at a decreased level in samples of plaque from risk subjects compared to samples from control subjects. Alternatively, a marker can be a polypeptide that is detected at a higher frequency or at a lower frequency in samples of plaque from risk subjects compared to samples from control subjects.

A polypeptide is "differentially present" between two samples if the amount of the polypeptide in one sample is statistically significantly different from the amount of the polypeptide in the other sample. For example, a polypeptide is differentially present between two samples if it is present at least about 120%, at least about 130%, at least about 150%, at least about 180%, at least about 200%, at least about 300%, at least about 500%, at least about 700%, at least about 900%, or at least about 1000% greater than it is present in the other sample, or if it is detectable in one sample and not detectable in the other.

The term "plaque" as used herein, is defined as a fatty build-up in the inner lining of blood vessel walls, composed of deposits of smooth muscle cells, fatty substances, cholesterol, calcium, and cellular waste products, usually associated with atherosclerosis. Build-up of plaque narrows the blood vessels and can change blood flow or completely block the flow of blood to essential organs, such as heart or brain. The term is used herein interchangeably with the term "atherosclerotic plaque". A "blood vessel" is preferably an artery, more preferably a carotid artery.

As used herein, the terms "antibody" and "antibodies" refer to monoclonal antibodies, multispecific antibodies, synthetic antibodies, human antibodies, humanized antibodies, chimeric antibodies, single-chain Fvs (scFv), single chain antibodies, Fab fragments, F(ab') fragments, disulfide-linked Fvs (sdFv), and anti-idiotypic (anti-Id) antibodies (including, e.g., anti-Id antibodies to antibodies of the invention), and epitope-binding fragments of any of the above. In particular, antibodies of the present invention include immunoglobulin molecules and immunologically active portions of immunoglobulin molecules, i.e., molecules that contain an antigen binding site that immunospecifically binds to a polypeptide antigen encoded by a gene comprised in the genomic regions or affected by genetic transformations in the genomic regions listed in Table 1. The immunoglobulin molecules of the invention can be of any type (e.g., IgG, IgE, IgM, IgD, IgA and IgY), class (e.g., IgG₁, IgG₂, IgG₃, IgG₄, IgA₁ and IgA₂) or subclass of immunoglobulin molecule.

"Immunoassay" is an assay that uses an antibody to specifically bind an antigen (e.g., a marker). The immunoassay is characterized by the use of specific binding properties of a particular antibody to isolate, target, and/or quantify the antigen. A variety of immunoassay formats may be used to select antibodies specifically immunoreactive with a particular protein. For example, solid-phase ELISA immunoassays are routinely used to select antibodies specifically immunoreactive with a protein (see, e.g., Harlow & Lane, Antibodies, A Laboratory Manual (1988), for a description of immunoassay formats and conditions that can be used to determine specific immunoreactivity). Typically a specific or selective reaction will be at least twice background signal or noise and more typically more than 10 to 100 times background.

The phrase "specifically (or selectively) binds" to an antibody or specifically (or selectively) immunoreactive with", when referring to a protein or peptide, refers to a binding reaction that is determinative of the presence of the protein in a heterogeneous population of proteins and other biologics. Thus, under designated immunoassay conditions, the specified antibodies bind to a particular protein at least two times the background and do not substantially bind in a significant amount to other proteins present in the sample. Specific binding to an antibody under such conditions may require an antibody that is selected for its specificity for a particular protein.

The terms "affecting the expression" and "modulating the expression" of a protein or gene, as used herein, should be understood as regulating, controlling, blocking, inhibiting, stimulating, enhancing, activating, mimicking, bypassing, correcting, removing, and/or substituting said expression, in more general terms, intervening in said expression, for instance by affecting the expression of a gene encoding that protein.

The terms "subject" or "patient" are used interchangeably herein and include, but are not limited to, an organism; a mammal, including, e.g., a human, non-human primate, mouse, pig, cow, goat, cat, rabbit, rat, guinea pig, hamster, horse, monkey, sheep, or other non-human mammal; and a non-mammal, including, e.g., a non-mammalian vertebrate, such as a bird (e.g., a chicken or duck) or a fish, and a non-mammalian invertebrate.

### Description of the preferred embodiments

### Biomarkers

The present invention is based on typing of an atherosclerotic plaque, and the identification of biomarkers. At the early state of atherosclerosis, preintrusive wall thickening is accompanied with lumen dilation in order to preserve blood flow (compensatory enlargement). At the more advanced stage plaque growth induces luminal stenosis that compromises blood flow. Plaque rupture often occurs before a high grade stenosis is reached. Atherosclerosis is the result of an excessive, inflammatory-fibroproliferative response. In advanced atherosclerotic plaques, the interplay between different cell types and the extracellular matrix may ultimately result in an inflammatory proteolytic response leading to plaque destabilization and rupture.

The mechanisms of plaque growth and rupture are poorly understood but encompass systemic and local factors. Unstable plaques are characterized by an infiltrate of T cells and macrophages which can lead to plaque disruption and thrombosis due to protease activation and subsequent matrix degradation. Upon disruption, the highly thrombogenic plaque content is exposed to circulating blood, triggering platelet activation and thrombus formation. It is now well established that rupture and superimposed thrombus formation associated with the progression of atherosclerotic plaques contribute in a critical manner to lethality associated with the development of atherosclerotic lesions. Several animal models for atherosclerosis have been developed and although these models are very important in atherosclerosis research, the animal models have 2 major disadvantages:
1 Atherosclerotic plaques in animal models and humans have a different morphology.
2 An appropriate model for plaque rupture is lacking.

In order to screen for suitable molecular targets involved in plaque progression and subsequent plaque rupture, human atherosclerotic specimens have to be used as a starting point. Due to limited availability, research is mostly performed in a small sample of atherosclerotic tissues not taking into account numerous confounding factors.

Certain aspects of the present invention have become possible through the development of the so-called Athero-Express Biobank (Verhoeven, *et al*., Eur. J. Epid. **19**:1127-1133, 2004), a bank containing samples of atherosclerotic plaques of patients that underwent surgery upon carotid arteries, femoral arteries and abdominal aneurysms. All patients whose plaques have been included have been followed up for at least 3 years.

A large-scale analysis of peptides generated from plaque proteins revealed that several of them were differentially expressed in the group of patients that during follow up suffered a major cardiovascular event (risk patients) and those patients who did not (non-risk patients). Thus, using this biobank the present inventors were able to discover a number of biomarkers that were associated with an increased risk of suffering a cardiovascular event. The biomarkers provided by the present invention may take the form of single protein biomarkers that are differentially present in-risk subjects, or may take the form of unique expression levels or concentrations of combinations of proteins that are indicative, so-called differential protein (expression) profiles. In general terms, the biomarker comprises at least one protein.

The proteins that have been identified by the present inventors as candidate biomarkers are listed in Table I. Table I lists the proteins that are differentially expressed between risk and no-risk patients. In Table III it is indicated for these proteins whether they are overexpressed/present or underexpressed/absent in the plaques.

Since all of these proteins are in one way or another associated with an increased risk of suffering a cardiovascular event, the relationship between these various proteins was analysed by use of the software program Ingenuity® Pathways Analysis (IPA) version 5.0 (Ingenuity Systems, Inc. Redwood City, CA, USA). This analysis revealed that most proteins were interrelated with respect to their biological function in the cell. In all, a total of 8 distinct biomolecular networks were found based on the protein biomarkers identified. Apparently, the complete cascade of proteins that forms these networks is differentially expressed. It is therefore envisioned that every protein in these networks could function as a biomarker to discriminate between risk patients and non-risk patients.

A first network was identified based on the proteins ACTR2, CAPN2, CAST, FSCN1, HCLS1, HNRPA2B1, HNRPC, HSP90AA1, IGFBP7, MYLK, RAC1, S100A8, SERPINF1, SNCA, SPP1, SSB, STRIP1, UCHL1, and YWHAQ. Several other proteins are also part of this biomolecular network. These proteins also constitute candidate biomarkers associated with an increased risk of suffering a cardiovascular event. This and other networks as defined in more detail above are indicated herein as "Ingenuity® network". Thus, an Ingenuity® network as the term is used herein, is defined by a number of proteins, but may comprise additional proteins. These additional proteins and their association to an increased risk of suffering a cardiovascular event, are part of the present invention. The candidate biomarkers are indicated in Fig. 1, wherein the biomarkers identified as being differentially expressed are indicated in bold type-face, and further candidate biomarkers (e.g. 14-3-3, Akt, Ap1, Arp2/3, Calmodulin, Calpain, etc) are in normal type-face.

Further selection of the proteins was made on basis of their presence (and connection) in Ingenuity™ networks (see examples). The invention comprises the use of one or more of the proteins listed in Table I and preferably Table II, and more preferably one or more of the proteins of Table III as a marker to diagnose patients having atherosclerotic plaques by methods of the invention. On basis of the level of expression of said one or more proteins it can be predicted whether the patient has a high risk of a major cardiovascular event. Especially preferred is the use of osteopontin (or SPP1) as a marker to diagnose patients. Osteopontin (herein also referred to as OPN) was studied in great detail in the samples of plaque available in the Athero-Express Biobank and proved to be a very valuable biomarker.

Osteopontin (OPN), also known as early T lymphocyte activation 1 (Eta-1) or SPP1, is a secreted multifunctional glycoprotein. Its putative functions include roles in bone metabolism, immuneregulation, wound healing, cell survival, and tumor progression. Based on gene structure and chromosomal location, OPN is a member of the small integrin-binding ligand N-linked glycoprotein (SIBLING) family that also includes bone sialoprotein (BSP), dentin matrix protein 1 (DMP1), dentin sialophosphoprotein (DSPP), enamelin (ENAM), and matrix extracellular phosphoglycoprotein (MEPE). Human OPN cDNA encodes a 314 amino acid (aa) precursor protein with a predicted 16 aa signal peptide that is cleaved to yield the 298 aa mature protein. It is a highly acidic, multi-domain protein with a predicted molecular weight of approximately 33 kDa, although it may range up to 75 kDa due to extensive glycosylation and phosphorylation.

OPN is expressed mainly by bone, kidney, and epithelial tissues but can also be found in endometrial tissues, endothelial cells, T cells, macrophages, smooth muscle cells, and many tumor types. It is upregulated in tissues during several pathological processes including atherosclerosis, valve stenosis, myocardial infarction, and rheumatoid arthritis.

OPN is found in several biological fluids including human plasma, serum, breast milk, and urine, and is upregulated in certain cancers, fulminant hepatitis, tuberculosis, and autoimmune diseases such as multiple sclerosis and lupus erythematosus.

OPN activities are mediated by an array of receptors including many integrins and the hyaluronan receptor, CD44. The OPN protein contains multiple domains responsible for integrin binding. It has a typical Arg-Gly-Asp (RGD) site that binds several integrins of the αV class, α5β1, and α8β1, a lopinavir (LPV)-containing domain that binds α4β1, and an SVVYGLR site that binds α4β1, α4β7, and α9β1. OPN may also associate with variants of CD44, potentially with integrin cooperation, to stimulate cell migration. An intracellular pool of OPN may also enhance cell migration through interaction with CD44 and ERM (ezrin/radixin/moesin) proteins, and/or via the regulation of CD44 cell surface expression.

The activities of OPN and proteases are reciprocally modulated. Cleavage by thrombin, MMP-3, MMP-7, or MMP-12 can produce OPN fragments that have biological activity different from the whole protein. For instance, thrombin, MMP-3, or MMP-7 cleavage may enhance integrin-dependent cell adhesion and/or migration. In turn, OPN is shown to activate MMP-2 or -3 by mechanisms that may include conversion/activation of the proform or reactivation of TIMP-inhibited enzyme.

As the name implies, OPN may have a role in bone metabolism. In vitro, it stimulates the adhesion of osteoclasts to bone, and bone resorption is blocked by inhibition of this interaction. Knockout mice have outwardly normal bone development, but do exhibit deficient postnatal bone resorption in several contexts, supporting a role for OPN in osteoclast function. OPN may also contribute directly to the regulation of mineral crystal formation and growth. It binds hydroxyapatite and suppresses crystal formation both *in vitro* and in *vivo.* OPN is also a regulator of inflammation. Inflammatory mediators including LPS, NO, IL-1β, and TNF-α, stimulate OPN expression. OPN regulates macrophage differentiation and recruitment. It also functions as a chemotactic factor and co-stimulator of T cells and may act as a Th1 cytokine, stimulating IL-12 production.

OPN knockout mice exhibit deficient Th1 responses and are susceptible to bacterial and viral infection. In contrast, OPN may have context-dependent anti-inflammatory effects as well. For instance, it suppresses the release of several inflammatory mediators from osteoarthritic chondrocytes in vitro.

The role of OPN in arterial pathology has been studied in Osteopontin null and transgenic mice. While in the Osteopontin null mouse neointima formation is inhibited, the opposite is found in the transgenic mouse. In an atherosclerotic background, OPN deficiency reduces the development of atherosclerotic plaques and reduced plasma cholesterol levels, the latter suggesting a role for OPN in lipid metabolism. On the other hand, OPN transgenic mice develop early fatty streak lesions and atherosclerotic lesions while angiotensin-II accelerated atherosclerosis and aneurysm formation is attenuated in OPN null mice.

In vitro, OPN has been related to coagulation based on the binding of thrombin-cleaved OPN on the integrin receptor αVβ3 which is in accordance with a high neutrophil infiltration mediated by OPN. In rat smooth muscle cells that are prominent cells in the atherosclerotic plaque, it was shown that OPN expression was enhanced with high glucose suggesting a role of OPN in diabetic vascular complications.

In a rabbit model, OPN is associated with VEGF induced adventitial angiogenesis while OPN also has been described to expressed higher in human unstable macrophage-rich carotid plaques compared to stable non-symptomatic plaques.

Plasma OPN levels have been measured in patients groups with different entities of cardiovascular disease. This revealed that patients with unstable angina pectoris had a higher OPN plasma levels compared to the stable angina group and are related to the presence and extend of coronary artery disease.

In a rat hypertension model with end-organ damage, plasma OPN was identified as a possible marker for end-organ damage. OPN expression was reduced with the p38 MAPK inhibitor SB-239063AN. In a diabetic rat, aortic OPN mRNA levels were reduced by the PPARgamma ligand pioglitazone and in rat hearts during hypertrophy by the mineralocorticoid receptor antagonist eplerenone. Next to this angiotension receptor type I and II blockade has been described to reduce kidney OPN expression.

### Prognostic and diagnostic methods

In a method of the present invention for predicting the risk of a subject developing a medical condition, the biomarker may be detected in atherosclerotic plaque from a subject by *in vivo* or non-invasive methods or by *ex vivo* methods involving the removal of a plaque sample. "Detect" refers to identifying the presence, absence or amount of the object to be detected. Detection may comprise the demonstration of the presence, in absolute (e.g., µg/ml) terms or in relative terms (e.g., relative intensity of signals), or of the absence of the biomarker in (a sample of) atherosclerotic plaque of subject. Very suitable, the amount of the biomarker relative to another protein stably present in the subject, such as a household enzyme, may determined in order to detect the biomarker in a subject.

Non-invasive methods for detecting or measuring proteins in the body of a subject (*in vivo*) are well known to the artisan. Such methods may include MRI, ultrasound spectroscopy, Raman spectroscopy and/or infra red spectroscopy and generally involve the use of specific labels for detection of the proteins.

Similar methods may be employed when analysing plaque samples for the same purpose. However, in addition, *ex vivo* methods may be applied on samples that are obtained by invasive methods, and include the use of mass spectrometry, protein chip array analysis, antibody array analysis, and/or immunoassay analysis for detection and/or quantification of the proteins on plaque samples.

A plaque sample may be provided by removing a plaque sample from the blood vessel of a subject. Plaque may be removed from the blood vessel by clamping the vessel, cutting the plaque out, and closing the opening back up. Very suitably, the plaque samples may be provided by atherectomy. Atherectomy involves the use of a cutting device (a blade, or a whirling blade called a rotoblade, and occasionally a laser beam), often attached to a catheter, to remove the plaque buildup from the blood vessel wall. The blood vessel may be an artery, suitably a coronary artery or a carotid artery. After removal of the plaque sample, the plaque specimen is kept for subsequent protein measurement under conditions that avoid protein breakdown. Very suitably, for collection of clinical data and annotation of the protein make-up of the plaque to a specific clinical picture, patients are monitored for a significant period (1-10 years) following the plaque sampling procedure.

The methods of the present invention may in principal be performed on plaque from any blood vessel. In preferred embodiments the blood vessel is a coronary, femoral and/or carotid artery.

The biomarkers of the present invention may be used in the methods of the invention for prognostic diagnosis of a variety of medical conditions, including, but not limited to cardiovascular events. A cardiovascular event is mostly the result of stenosis. Stenosis, or the narrowing or blockage of an artery or other vessel due to plaque build-up may result in a large number of adverse conditions, many of which have severe consequences for the subject.

Based on the demonstration that plaque proteins are so closely associated to prognosis of certain diseases or medical conditions, the present invention now provides a method for predicting the risk of a test subject developing a medical condition comprising the steps of a) typing atherosclerotic plaque to identify a biomarker associated to the medical condition, and b) detecting said biomarker in (a sample of) atherosclerotic plaque from said test subject, wherein said test subject is at risk of developing said medical condition when said biomarker is present in said (sample of) atherosclerotic plaque.

The prognostically diagnosed medical condition includes the risk of suffering a cardiovascular event. In fact, by using this method, the skilled person will be able to find additional biomarkers for use in a method of the present invention.

Typing of atherosclerotic plaque involves taking a sample from a subject at risk of developing a medical condition, and a sample from a control subject not at risk of developing said condition. Whether a subject is at risk of developing a medical condition, is often revealed after a prolonged period of time, following the sampling of the plaque. For instance, the plaque may have the protein constitution or protein profile of a type associated with increased risk (for instance as predicted from database records), yet, it may take many years before said risk materializes in the form of, for instance, a cardiovascular event. Alternatively, when a subject has developed a medical condition, for instance, has suffered a stroke, then it is immediately clear that the subject is a test subject at risk of developing a medical condition, and a follow-up period is not essential to derive the relevant clinical information and to annotate the relevant clinical information to the plaque sample. Preferably, the collection of information on the medical condition of the subjects donating the plaque is performed during a follow-up period, preferably of about 0.1-80, more preferably of about 1-10 years.

Typing of a plaque sample in a method of the invention further comprises the step of measuring the amount of at least one protein in a positive control plaque (from a risk patient) and in a negative control plaque (from a non-risk patient) or providing a protein profile for both samples. The term "amount of at least one protein" as used in this description, may refer to a relative amount or an absolute amount (e.g. a concentration). A positive control plaque is also referred to as a reference sample and the amount of protein therein is referred to as the reference value (i.e. above or below which there is a positive identification of the presence of a risk). A negative control plaque is also referred to herein as a control sample.

It will be appreciated that the step of measuring the amount of at least one protein need not result in an exact determination of the concentration of protein in said sample. It is sufficient that an expression of the amount is obtained relative to the amount present (or not present) in a control sample. Any (semi) quantitative method is suitable, as long as the measured amount can be compared with control or reference values.

In order to identify a candidate biomarker, typing includes the step of determining whether said at least one protein is differentially present in said first (sample of) atherosclerotic plaque compared to said second (sample of) atherosclerotic plaque, or determining the differential protein expression profile between said first and second (sample of) atherosclerotic plaque. This step may conveniently be performed by using protein expression arrays, or by analysing the proteins present in the two plaque sample by 2-dimensional poly-acrylamide gel electrophoreses (2-D PAGE) and western blotting or mass spectrometry. Such methods generally involve the partial degradation of the proteins into peptides and the sequencing and subsequent identification of these peptides by tandem-MS. Such methods are well established (see: Principles of Proteomics by R. M. Twyman BIOS Scientific Publ; 1st edition, September 24, 2004)

When the differential expression profile is determined, and the amount(s) of proteins present in the samples that resemble the risk and non-risk condition are determined, the amounts much be correlated to the condition. Statistical analysis thereof involves routine procedures, provided that clinical data for the medical condition under study are properly annotated to the samples analysed.

Finally, the differentially present protein or differential protein expression profile is identified as a biomarker when there is indeed a correlation between the occurrence of the medical condition and the presence (or absence) of the biomarker.

The present invention also provides a kit of parts for performing the methods as described above. Such kits of parts are based on the detection of the biomarker by *in vivo* or *ex vivo* methods as described above. A kit of parts of the present invention comprises a biomarker, or a detectable binding partner thereof, for instance an antibody that binds specifically to the biomarker.

A kit of parts may further comprise components for validating the detection protocol, such as reference or control samples, information on the reference value for the biomarker, peptides capable of binding to the antibody and which can for instance be used in competitive ELISA assays; detectable markers, often containing a labelling moiety, for detecting binding between said biomarker and said antibody.

Labelling moieties may include fluorescent, chemiluminescent, magnetic, radioactive or other moieties suitable for detection by dedicated equipment

The measured concentration may then be compared to reference values available in a database. Such a database may have the form of a listing of proteins, preferably plaque proteins, wherein to each protein is annotated a reference or threshold value below or above which the risk on the occurrence of a cardiovascular event in a patient is increased. In order to determine the threshold value for each protein, a comprehensive study may be performed between samples from risk-patients (patients that have suffered a cardiovascular event) and non-risk patients (that have not suffered from a cardiovascular event), e.g. such as described herein, and wherein the threshold value is the uppermost or lowest value among the non-risk patients, above which, respectively, below which the statistical chance on the occurrence of a cardiovascular event is significantly increased. The study as described herein made use of the so-called Athero-Express Biobank, a bank containing samples of atherosclerotic plaques of patients that underwent surgery upon carotid arteries, femoral arteries and abdominal aneurysms, and for which patients the occurrence of a cardiovascular event was recorded during a period of three years following the surgery.

Alternatively, the database may take the form of a collection of one or more reference samples, containing the said at least one protein in an amount equal to the reference value for that protein. In such instances, the steps of measuring the amount of at least one protein in a sample and the step of comparing the measured amount with reference values, may be performed in a single assay wherein the amount of said protein in test and control sample is determined relative to each other, for instance by using any available differential expression analysis technique. Any method suitable for analysing the differential expression of proteins between samples may be used in such instance. When the differential expression of a large number of proteins is required, antibody microarrays may suitably be used may be used.

The preparation of antibody microarray on e.g. glass slides is known to the skilled person. Antibodies may be spotted on for instance amino-reactive glass slides or other functionalized surfaces. Generally, methods are available to the skilled person to print as many as 20000 spots on a single 2.5 x 7.5 cm glass slide with individual spots being spotted about 300 µm apart. In order to allow the performance of multiple binding experiments on a single slide, a number of grids consisting of a defined group of antibodies can be spotted on one slide. The antibodies may be spotted by any available spotting technique, for instance by contact printing. Tools and technologies developed for the production of DNA microarrays, such as spotter, incubation chambers, differential fluorescent labelling techniques and imaging equipment for quantitative measurement of binding studies, are readily available to the artisan.

In order to prepare antibody microarrays, it will be appreciated that the availability of the purified proteins is not required. By using methods such as peptide immunisation, information on the protein or peptide sequences (e.g. deduced from genes identified in transcriptional studies) is sufficient to design peptides, which upon synthesis and injection into rabbits can be used to raise antibodies. Such antibodies may be used to measure the amount of the native protein in biological samples. After affinity purification, these antibodies may then be used for the preparation of antibody arrays as described above. Procedures for the preparation of antibody arrays based on protein or peptide sequences are commercially available, for instance from Eurogentec, Seraing, Belgium.

As stated, the antibody microarrays may be used for differential protein expression studies (protein profiling). In order to measure the differential expression of proteins in a biological sample under an experimental condition and compare the expression with control samples or reference values, several methods may be used for labelling of the proteins. Very suitable, the proteins from the biological samples are labelled with one or more fluorescent probes (e.g. Cy3 and Cy5) using standard protein labelling protocols. Once the proteins of a biological sample (test and control) have been labelled (preferably differentially labelled using different colour probes for test and control), they can be brought in contact with the antibody microarray. The binding of the antigens to the antibody array may for instance be performed upon incubation of the microarray slide with a small volume (± 50 µl) of labelled biological material, under cover slips. The detection of protein bound to the antibody microarray may be based on the generation of fluorescence. Proteins that bind to the microarray may then be detected using a fluorescent scanner and individual spots of the chip can then be analysed to determine the differential expression between the test and control sample.

In alternative procedures, the antibody microarrays may be used as capturing chips for the quantification of multiple proteins in a biological sample using ELISA methods on the chip. The various proteins identified as biomarkers for assessing the risk of a cardiovascular event as described herein may be measured more quantitatively by such procedures. To determine the concentration of a protein in a biological sample, ELISA techniques are very suitable. Such techniques involve the production of a calibration curve of the fluorescence intensity vs. protein concentration, or the use of a competitive ELISA format, wherein known amounts of unlabelled protein or antigen are provided in the test. When using methods such as peptide immunisation for the preparation of an antibody microarrays as described above, the peptides used for immunisation may be used in competitive ELISA experiments on the microarray. Alternatively, multiple sandwich ELISA can be developed using as second antibody, for instance an antibody raised by peptide immunisation against a second epitope of the target protein (a second synthetic peptide).

In yet another aspect, the present invention provides the use of a biomarker as defined herein above for predicting the risk of a cardiovascular event in a subject. Such use involves the detection of the biomarker in (a sample of) atherosclerotic plague, and the determination whether the amount detected is above or below the reference value.

### Therapeutic methods

In yet another aspect, the present invention provides a method of treating a subject having an increased risk of suffering a cardiovascular event, said method comprising using a biomarker as defined herein above as a therapeutic target or as a therapeutic agent. Preferably, said use of said biomarker as a therapeutic target comprises decreasing the amount of at least one protein that is over-expressed in subjects having an increased risk of suffering a cardiovascular event, or increasing the amount of at least one protein that is under-expressed in subjects having an increased risk of suffering a cardiovascular event. More preferably said protein that is over-expressed is SPP1 (osteopontin).

Preferably, said use of said biomarker as a therapeutic agent comprises increasing the amount of at least one protein that is under-expressed in subjects having an increased risk of suffering a cardiovascular event, and involves administrating said protein to said subject.

The present invention also relates to the use of the biomarkers of the present invention as therapeutic targets. Pharmacogenetics and pharmacogenomics aim at determining the genetic determinants linked to diseases. Most of the diseases are multigenic diseases, and the identification of the genes involved therein should allow for the discovery of new targets and the development of new drugs.

Many physiological diseases are targeted by this novel pharmaceutical approach. One can name the autoimmune and inflammatory diseases, for example Addison's disease, Alopecia Areata, Ankylosing Spondylitis, Behcet's Disease, Chronic Fatigue Syndrome, Crohn's disease, Ulcerative Colitis, Inflammatory Bowel disease, Diabetes and Multiple Sclerosis.

The risk of suffering a cardiovascular event due to a certain constitution of atherosclerotic plaque may be viewed as a multigenic disease. The biomarkers of the present invention have been identified as genetic markers for predisposition of the disease. In fact, not all plaques are equally dangerous, but the risk level of the plaque is associated with the occurrence therein (or non-occurrence therein) of specific proteins. The identification of these proteins and their associated genes provides better information of the patient and allows for the prevention of the development of the disease itself and an improved life expectancy of the patient.

Knowledge of the identity of genes involved in plaque development ultimately resulting in a fatal cardiovascular event therefore greatly facilitates the development of prophylactic, therapeutic and diagnostic methods for this disease. Diagnosis of the genes responsible for the risk phenotype in a certain subjects allows for the design of therapies comprising the use of specific drugs, for instance, drugs directed against the proteins encoded by these genes.

It is an aspect of the present invention to use the protein markers of the present invention and/or the genes encoding these proteins for the development of inhibitors directed against the genes and/or their expression products (RNA or protein), in particular in the case of over-expression of the biomarker in the subject at risk.

In one embodiment of this aspect, the inhibitors are antibodies and/or antibody derivatives directed against the expression products of genes encoding the biomarkers. Therapeutic antibodies are for instance useful against gene expression products located on the cellular membrane and can be comprised in a pharmaceutical composition. Also, antibodies may be targeted to intracellular, e.g. cytoplasmic, gene products such as RNA's, polypeptides or enzymes, in order to modulate the activity of these products. Preferably, such antibodies are in the form of intrabodies, produced inside a target cell, preferably a plaque-forming cell including T-cells, endothelial cells, and smooth muscle cells, or cells that are found in atherosclerotic lesions, such as leukocytes, macrophages, foam cells, dendritic cells, and mast cells and T cells. In addition, antibodies may be used for deliverance of at least one toxic compound linked thereto to a target cell.

In a preferred embodiment of the present invention, the inhibitor is a small molecule capable of modulating the activity or interfering with the function of the protein expression product of the genes encoding the biomarkers as defined herein. In addition, small molecules can also be used for deliverance of at least one linked toxic compound to the target cell.

On a different level of inhibition, nucleic acids can be used to block the production of proteins by destroying the mRNA transcribed from respective gene encoding the biomarkers. This can be achieved by antisense drugs, ribozymes or by RNA interference (RNAi). By acting at this early stage in the disease process, these drugs prevent the production of a disease-causing protein. The present invention relates to antisense drugs, such as antisense RNA and antisense oligodeoxynucleotides, ribozymes and RNAi molecules, directed against the genes encoding the boiomarkers.

The expression level of a gene can either be decreased or increased in a risk phenotype. Naturally, inhibitors are used when the expression levels are elevated. However, the present invention also provides for "enhancers", to boost the expression level of a gene encoding the biomarkers associated with a risk of suffering a cardiovascular event and of which the expression levels are reduced in a risk situation. "Enhancers" may be any chemical or biological compound known or found to increase the expression level of genes, to improve the function of an expression product of a gene or to improve or restore the expression of a gene.

Very suitable therapies to overcome reduced expression levels of a gene or to restore the expression of a gene encoding the biomarkers as disclosed herein include the replacement by gene therapy of the gene or its regulatory sequences that drive the expression of said gene. The invention therefore relates further to gene therapy, in which a dysfunctional gene of a subject encoding the biomarkers or a dysfunctional regulatory sequence of a gene of a subject encoding a biomarker is replaced by a functional counterpart, e.g. by stable integration of for instance a lentiviral vector comprising a functional gene or regulatory sequence into the genome of a subject's host cell which is a progenitor cell of the target cell-line of the subject and grafting of said transfected host cell into said subject.

The invention also relates to forms of gene therapy, in which the genes encoding the biomarker are i.a. used for the design of dominant-negative forms of these genes which inhibit the function of their wild-type counterparts following their directed expression from a suitable vector in a target cell.

Another object of the present invention is to provide a pharmaceutical composition for the treatment of patients having an increased risk of suffering a cardiovascular event comprising one or more of the inhibitors, "enhancers", replacement compounds, vectors or host cells according to the present invention as a pharmaceutical reagent or active ingredient. The composition can further comprise at least one pharmaceutical acceptable additive like for example a carrier, an emulsifier, or a conservative.

In addition, it is the object of the present invention to provide a method for treatment of subjects suffering from an increased risk of suffering a cardiovascular event which method comprises the administration of the pharmaceutical composition according to the invention to patients in need thereof in a therapeutically effective amount.

### Small molecule inhibitors

Small molecule inhibitors are usually chemical entities that can be obtained by screening of already existing libraries of compounds or by designing compounds based on the structure of the protein encoded by a gene involved in tumor development. Briefly, the structure of at least a fragment of the protein is determined by either Nuclear Magnetic Resonance or X-ray crystallography. Based on this structure, a virtual screening of compounds is performed. The selected compounds are synthesized using medicinal and/or combinatorial chemistry and thereafter analyzed for their inhibitory effect on the protein *in vitro* and in *vivo.* This step can be repeated until a compound is selected with the desired inhibitory effect. After optimization of the compound, its toxicity profile and efficacy as therapeutic is tested *in vivo* using appropriate animal model systems.

Differentially expressed genes that do not encode membrane-bound proteins are selected as targets for the development of small molecule inhibitors. To identify putative binding sites or pockets for small molecules on the surface of the target proteins, the three-dimensional structure of those targets are determined by standard crystallization techniques (de Vos et al. 1988, Science 239:888-93; Williams et al. 2001, Nat Struct Biol 8:838-42). Additional mutational analysis may be performed to confirm the functional importance of the identified binding sites. Subsequently, Cerius2 (Molecular Simulations Inc., San Diego, CA, USA) and Ludi/ACD (Accelrys Inc., San Diego, CA, USA) software is used for virtual screening of small molecule libraries (Bohm. 1992 J Comp Aided Molec Design 6:61-78). The compounds identified as potential binders by these programs are synthesized by combinatorial chemistry and screened for binding affinity to the targets as well as for their inhibitory capacities of the target protein's function by standard *in vitro* and *in vivo* assays. In addition to the rational development of novel small molecules, existing small molecule compound libraries are screened using these assays to generate lead compounds. Lead compounds identified are subsequently co-crystallized with the target to obtain information on how the binding of the small molecule can be improved (Zeslawska et al. 2000, J Mol Biol 301:465-75). Based on these findings, novel compounds are designed, synthesized, tested, and co-crystallized. This optimization process is repeated for several rounds leading to the development of a high-affinity compound of the invention that successfully inhibits the function of its target protein. Finally, the toxicity of the compound is tested using standard assays (commercially available service via MDS Pharma Services, Montreal, Quebec, Canada) after which it is screened in an animal model system.

### Ribozymes

Trans-cleaving catalytic RNAs (ribozymes) are RNA molecules possessing endoribonuclease activity. Ribozymes are specifically designed for a particular target, and the target message must contain a specific nucleotide sequence. They are engineered to cleave any RNA species site-specifically in the background of cellular RNA. The cleavage event renders the mRNA unstable and prevents protein expression. Importantly, ribozymes can be used to inhibit expression of a gene of unknown function for the purpose of determining its function in an in vitro or in vivo context, by detecting the phenotypic effect.

One commonly used ribozyme motif is the hammerhead, for which the substrate sequence requirements are minimal. Design of the hammerhead ribozyme is disclosed in Usman et al., Current Opin. Struct. Biol. (1996) 6:527-533. Usman also discusses the therapeutic uses of ribozymes. Ribozymes can also be prepared and used as described in Long et al., FASEB J. (1993) 7:25; Symons, Ann. Rev. Biochem. (1992) 61:641; Perrotta et al., Biochem. (1992) 31:16-17; Ojwang et al., Proc. Natl. Acad. Sci. (USA) (1992) 89:10802-10806; and U.S. Pat. No. 5,254,678. Ribozyme cleavage of HIV-I RNA is described in U.S. Pat. No. 5,144,019; methods of cleaving RNA using ribozymes is described in U.S. Pat. No. 5,116,742; and methods for increasing the specificity of ribozymes are described in U.S. Pat. No. 5,225,337 and Koizumi et al., Nucleic Acid Res. (1989) 17:7059-707L Preparation and use of ribozyme fragments in a hammerhead structure are also described by Koizumi et al., Nucleic Acids Res. (1989) 17:7059-7071. Preparation and use of ribozyme fragments in a hairpin structure are described by Chowrira and Burke, Nucleic Acids Res. (1992) 20:2835. Ribozymes can also be made by rolling transcription as described in Daubendiek and Kool, Nat. Biotechnol. (1997) 15(3):273-277.

The hybridizing region of the ribozyme may be modified or may be prepared as a branched structure as described in Horn and Urdea, Nucleic Acids Res. (1989) 17:6959-67. The basic structure of the ribozymes may also be chemically altered in ways familiar to those skilled in the art, and chemically synthesized ribozymes can be administered as synthetic oligonucleotide derivatives modified by monomeric units. In a therapeutic context, liposome mediated delivery of ribozymes improves cellular uptake, as described in Birikh et al., Eur. J. Biochem. (1997) 245:1-16.

Therapeutic and functional genomic applications of ribozymes proceed beginning with knowledge of a portion of the coding sequence of the gene to be inhibited. Thus, for many genes, a nucleic acid sequence provides adequate sequence for constructing an effective ribozyme. A target cleavage site is selected in the target sequence, and a ribozyme is constructed based on the 5' and 3' nucleotide sequences that flank the cleavage site. Retroviral vectors are engineered to express monomeric and multimeric hammerhead ribozymes targeting the mRNA of the target coding sequence. These monomeric and multimeric ribozymes are tested in vitro for an ability to cleave the target mRNA. A cell line is stably transduced with the retroviral vectors expressing the ribozymes, and the transduction is confirmed by Northern blot analysis and reverse-transcription polymerase chain reaction (RT-PCR). The cells are screened for inactivation of the target mRNA by such indicators as reduction of expression of disease markers or reduction of the gene product of the target mRNA.

### Antisense

Antisense polynucleotides are designed to specifically bind to RNA, resulting in the formation of RNA-DNA or RNA-RNA hybrids, with an arrest of DNA replication, reverse transcription or messenger RNA translation. Antisense polynucleotides based on a selected sequence can interfere with expression of the corresponding gene.

Antisense polynucleotides are typically generated within the cell by expression from antisense constructs that contain the antisense strand as the transcribed strand. Antisense polynucleotides will bind and/or interfere with the translation of the corresponding mRNA. As such, antisense may be used therapeutically the inhibit the expression of oncogenes.

Antisense RNA or antisense oligodeoxynucleotides (antisense ODNs) can both be used and may also be prepared *in vitro* synthetically or by means of recombinant DNA techniques. Both methods are well within the reach of the person skilled in the art. ODNs are smaller than complete antisense RNAs and have therefore the advantage that they can more easily enter the target cell. In order to avoid their digestion by DNAse, ODNs and antisense RNAs may be chemically modified. For targeting to the desired target cells, the molecules may be linked to ligands of receptors found on the target cells or to antibodies directed against molecules on the surface of the target cells.

### RNAi

RNAi refers to the introduction of homologous double stranded RNA to specifically target the transcription product of a gene, resulting in a null or hypomorphic phenotype. RNA interference requires an initiation step and an effector step. In the first step, input double-stranded (ds) RNA is processed into nucleotide 'guide sequences'. These may be single- or double-stranded. The guide RNAs are incorporated into a nuclease complex, called the RNA-induced silencing complex (RISC), which acts in the second effector step to destroy mRNAs that are recognized by the guide RNAs through base-pairing interactions. RNAi molecules are thus double stranded RNAs (dsRNAs) that are very potent in silencing the expression of the target gene. The invention provides dsRNAs complementary to the genes encoding the biomarkers of the present invention.

The ability of dsRNA to suppress the expression of a gene corresponding to its own sequence is also called post-transcriptional gene silencing or PTGS. The only RNA molecules normally found in the cytoplasm of a cell are molecules of single-stranded mRNA. If the cell finds molecules of double-stranded RNA, dsRNA, it uses an enzyme to cut them into fragments containing in general 21-base pairs (about 2 turns of a double helix). The two strands of each fragment then separate enough to expose the antisense strand so that it can bind to the complementary sense sequence on a molecule of mRNA. This triggers cutting the mRNA in that region thus destroying its ability to be translated into a polypeptide. Introducing dsRNA corresponding to a particular gene will knock out the cell's endogenous expression of that gene. This can be done in particular tissues at a chosen time. A possible disadvantage of simply introducing dsRNA fragments into a cell is that gene expression is only temporarily reduced. However, a more permanent solution is provided by introducing into the cells a DNA vector that can continuously synthesize a dsRNA corresponding to the gene to be suppressed.

RNAi molecules are prepared by methods well known to the person skilled in the art. In general an isolated nucleic acid sequence comprising a nucleotide sequence which is substantially homologous to the sequence of at least one of the genes encoding the biomarkers of the invention and which is capable of forming one or more transcripts able to form a partially of fully double stranded (ds) RNA with (part of) the transcription product of said genes will function as an RNAi molecule. The double stranded region may be in the order of between 10-250, preferably 10-100, more preferably 20-50 nucleotides in length.

RNAi molecules are preferably expressed from recombinant vectors in transduced host cells, hematopoietic stem cells being very suitable thereto.

### Dominant Negative Mutations

Dominant negative mutations are readily generated for corresponding proteins that are active as multimers. A mutant polypeptide will interact with wild-type polypeptides (made from the other allele) and form a non-functional multimer. Thus, a mutation is in a substrate-binding domain, a catalytic domain, or a cellular localization domain. Preferably, the mutant polypeptide will be overproduced. Point mutations are made that have such an effect. In addition, fusion of different polypeptides of various lengths to the terminus of a protein can yield dominant negative mutants. General strategies are available for making dominant negative mutants. See Herskowitz, Nature (1987) 329:219-222. Such a technique can be used for creating a loss of function mutation, which is useful for determining the function of a protein.

### Use of Polypeptides to raise Antibodies

The present invention provides in one aspect for antibodies suitable for therapeutic and/or diagnostic use.

Therapeutic antibodies include antibodies that can bind specifically to the expression products of the genes encoding the biomarkers of the invention. By binding directly to the gene products, the antibodies may influence the function of their targets by, for example, in the case of proteins, steric hindrance, or by blocking at least one of the functional domains of those proteins. As such, these antibodies may be used as inhibitors of the function of the gene product. Such antibodies may for instance be generated against functionally relevant domains of the proteins and subsequently screened for their ability to interfere with the target's function using standard techniques and assays (see for instance Schwartzberg, 2001, Crit Rev Oncol Hematol 40:17-24; Herbst et al. Cancer 94:1593-611, 2002).

Alternatively, anti-RNA antibodies may for instance be useful in silencing messengers of the tumor-related genes of the present invention. In another alternative, antibodies may also be used to influence the function of their targets indirectly, for instance by binding to members of signaling pathways in order to influence the function of the targeted proteins or nucleic acids. In yet another alternative, therapeutic antibodies may carry one or more toxic compounds that exert their effect on the target or target cell by virtue of the binding of the carrying antibody thereto.

For diagnostic purposes, antibodies similar to those above, preferably those that are capable of binding to the expression products of the genes of the present invention may be used, and that are provided with detectable labels such as fluorescent, luminescent, or radio-isotope labels in order to allow the detection of the gene product. Preferably such diagnostic antibodies are targeted to proteinaceous targets present on the outer envelop of the cell, such as membrane bound target proteins (biomarkers).

The antibodies used in the present invention may be from any animal origin including birds and mammals (*e.g*., human, murine, donkey, sheep, rabbit, goat, guinea pig, camel, horse, or chicken). Preferably, the antibodies of the invention are human or humanized monoclonal antibodies. As used herein, "human" antibodies include antibodies having the amino acid sequence of a human immunoglobulin and include antibodies isolated from human immunoglobulin libraries (including, but not limited to, synthetic libraries of immunoglobulin sequences homologous to human immunoglobulin sequences) or from mice that express antibodies from human genes.

For some uses, including *in vivo* therapeutic or diagnostic use of antibodies in humans and *in vitro* detection assays, it may be preferred to use human or chimeric antibodies. Completely human antibodies are particularly desirable for therapeutic treatment of human subjects. Human antibodies can be made by a variety of methods known in the art including phage display methods described above using antibody libraries derived from human immunoglobulin sequences or synthetic sequences homologous to human immunoglobulin sequences. See also U.S. Patent Nos. 4,444,887 and 4,716,111; and PCT publications WO 98/46645, WO 98/50433, WO 98/24893 and WO98/16654, each of which is incorporated herein by reference in its entirety.

The antibodies to be used with the methods of the invention include derivatives that are modified, *i.e*, by the covalent attachment of any type of molecule to the antibody such that covalent attachment. Additionally, the derivative may contain one or more non-classical amino acids.

In certain embodiments of the invention, the antibodies to be used with the invention have extended half-lives in a mammal, preferably a human, when compared to unmodified antibodies. Antibodies or antigen-binding fragments thereof having increased *in vivo* half-lives can be generated by techniques known to those of skill in the art (see, *e.g.,* PCT Publication No. WO 97/34631).

In certain embodiments, antibodies to be used with the methods of the invention are single-chain antibodies. The design and construction of a single-chain antibody is described in Marasco et al, 1993, Proc Natl Acad Sci 90:7889-7893, which is incorporated herein by reference in its entirety.

In certain embodiments, the antibodies to be used with the invention bind to an intracellular epitope, *i.e*., are intrabodies. An intrabody comprises at least a portion of an antibody that is capable of immunospecifically binding an antigen and preferably does not contain sequences coding for its secretion. Such antibodies will bind its antigen intracellularly. In one embodiment, the intrabody comprises a single-chain Fv ("sFv"). For a review of sFv see Pluckthun in The Pharmacology of Monoclonal Antibodies, vol. 113, Rosenburg and Moore eds. Springer-Verlag, New York, pp. 269-315 (1994). In a further embodiment, the intrabody preferably does not encode an operable secretory sequence and thus remains within the cell (see generally Marasco, WA, 1998, "Intrabodies: Basic Research and Clinical Gene Therapy Applications" Springer:New York).

Generation of intrabodies is well-known to the skilled artisan and is described for example in U.S. Patent Nos. 6,004,940; 6,072,036; 5,965,371, which are incorporated by reference in their entireties herein.

In one embodiment, intrabodies are expressed in the cytoplasm. In other embodiments, the intrabodies are localized to various intracellular locations. In such embodiments, specific localization sequences can be attached to the intranucleotide polypepetide to direct the intrabody to a specific location.

The antibodies to be used with the methods of the invention or fragments thereof can be produced by any method known in the art for the synthesis of antibodies, in particular, by chemical synthesis or preferably, by recombinant expression techniques.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981) (said references incorporated by reference in their entireties). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Examples of phage display methods that can be used to make the antibodies of the present invention include those disclosed in WO97/13844; and U.S. Patent Nos. 5,580,717, 5,821,047, 5,571,698, 5,780,225, and 5,969,108; each of which is incorporated herein by reference in its entirety.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, *e.g.,* as described below. Techniques to recombinantly produce Fab, Fab' and F(ab')2 fragments can also be employed using methods known in the art such as those disclosed in PCT publication No. WO 92/22324; Mullinax et al., 1992, BioTechniques 12(6):864-869 and Better et al., 1988, Science 240:1041-1043.

It is also possible to produce therapeutically useful IgG, IgA, IgM and IgE antibodies. For an overview of the technology for producing human antibodies, see Lonberg and Huszar (1995, Int. Rev. Immunol. 13:65-93). For a detailed discussion of the technology for producing human antibodies and human monoclonal antibodies and protocols for producing such antibodies, see, *e.g*., PCT publication No. WO 98/24893, which is incorporated by reference herein in its entirety. In addition, companies such as Medarex, Inc. (Princeton, NJ), Abgenix, Inc. (Freemont, CA) and Genpharm (San Jose, CA) can be engaged to provide human antibodies directed against a selected antigen using technology similar to that described above.

Recombinant expression used to produce the antibodies, derivatives or analogs thereof (*e.g*., a heavy or light chain of an antibody of the invention or a portion thereof or a single chain antibody of the invention), requires construction of an expression vector containing a polynucleotide that encodes the antibody and the expression of said vector in a suitable host cell or even *in vivo.* Once a polynucleotide encoding an antibody molecule or a heavy or light chain of an antibody, or portion thereof (preferably, but not necessarily, containing the heavy or light chain variable domain), of the invention has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an antibody molecule of the invention, a heavy or light chain of an antibody, a heavy or light chain variable domain of an antibody or a portion thereof, or a heavy or light chain CDR, operably linked to a promoter. Such vectors may include the nucleotide sequence encoding the constant region of the antibody molecule (see, e.g., PCT Publication WO 86/05807; PCT Publication WO 89/01036; and U.S. Patent No. 5,122,464) and the variable domain of the antibody may be cloned into such a vector for expression of the entire heavy, the entire light chain, or both the entire heavy and light chains.

The expression vector is transferred to a host cell by conventional techniques and the transfected cells are then cultured by conventional techniques to produce an antibody of the invention. Thus, the invention includes host cells containing a polynucleotide encoding an antibody of the invention or fragments thereof, or a heavy or light chain thereof, or portion thereof, or a single chain antibody of the invention, operably linked to a heterologous promoter. In preferred embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibody molecules as defined herein

In mammalian host cells, a number of viral-based expression systems may be utilized. In cases where an adenovirus is used as an expression vector, the antibody coding sequence of interest may be ligated to an adenovirus transcription/translation control complex, *e.g.,* the late promoter and tripartite leader sequence. This chimeric gene may then be inserted in the adenovirus genome by *in vitro* or *in vivo* recombination. Insertion in a non-essential region of the viral genome (*e.g*., region E1 or E3) will result in a recombinant virus that is viable and capable of expressing the antibody molecule in infected hosts (*e.g*., see Logan & Shenk, 1984, Proc. Natl. Acad. Sci. USA 81:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see, *e.g*., Bittner et al., 1987, Methods in Enzymol. 153:516-544).

Once an antibody molecule to be used with the methods of the invention has been produced by recombinant expression, it may be purified by any method known in the art for purification of an immunoglobulin molecule, for example, by chromatography (*e.g*., ion exchange, affinity, particularly by affinity for the specific antigen after Protein A, and sizing column chromatography), centrifugation, differential solubility, or by any other standard technique for the purification of proteins. Further, the antibodies of the present invention or fragments thereof may be fused to heterologous polypeptide sequences described herein or otherwise known in the art to facilitate purification.

As stated above, according to a further aspect, the invention provides an antibody as defined above for use in therapy.

For therapeutic treatment, antibodies may be produced *in vitro* and applied to the subject in need thereof. The antibodies may be administered to a subject by any suitable route, preferably in the form of a pharmaceutical composition adapted to such a route and in a dosage which is effective for the intended treatment. Therapeutically effective dosages of the antibodies required for decreasing the rate of progress of the disease or for eliminating the disease condition can easily be determined by the skilled person.

Alternatively, antibodies may be produced by the subject itself by using *in vivo* antibody production methodologies as described above. Suitably, the vector used for such *in vivo* production is a viral vector, preferably a viral vector with a target cell selectivity for specific target cell referred to herein.

Therefore, according to a still further aspect, the invention provides the use of an antibody as defined above in the manufacture of a medicament for use in the treatment of a subject to achieve the said therapeutic effect. The treatment comprises the administration of the medicament in a dose sufficient to achieve the desired therapeutic effect. The treatment may comprise the repeated administration of the antibody.

According to a still further aspect, the invention provides a method of treatment of a human comprising the administration of an antibody as defined above in a dose sufficient to achieve the desired therapeutic effect, wherein the therapeutic effect is the alleviation or prevention of the risk of suffering a cardiovascular event.

The diagnostic and therapeutic antibodies are preferably used in their respective application for the targeting of kinases or phosphatases, which are often coupled to receptor molecules on the cell's surface. As such, antibodies capable of binding to these receptor molecules can exert their activity-modulating effect on the kinases or phosphatases by binding to the respective receptors. Also transporter proteins may be targeted with advantage for the same reason that the antibodies will be able to exert their activity-modulating effect when present extracellularly. The above targets, together with signaling molecules, represent preferred targets for the antibody uses of the invention as more effective therapy and easier diagnosis is possibly thereby.

The diagnostic antibodies can suitably be used for the qualitative and quantitative detection of gene products, preferably proteins in assays for the determination of altered levels of proteins or structural changes therein. Protein levels may for instance be determined in cells, in cell extracts, in supernatants, body fluids by for instance flow-cytometric evaluation of immunostained target cells, preferably in plaque. Alternatively, quantitative protein assays such as ELISA or RIA, Western blotting, and imaging technology (e.g., using confocal laser scanning microscopy) may be used in concert with the antibodies as described herein for the diagnosis of an increased risk on cardiovascular events.

### Pharmaceutical Compositions and Therapeutic Uses

Pharmaceutical compositions can comprise polypeptides, antibodies, polynucleotides (antisense, RNAi, ribozyme), or small molecules of the claimed invention, collectively called inhibitor compounds herein. The pharmaceutical compositions will comprise a therapeutically effective amount of either a biomarker protein, an antibody, a polynucleotides or small molecules as described herein.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician. Specifically, the compositions of the present invention can be used to treat, ameliorate, or prevent the occurrence of a cardiovascular event in a subject and/or accompanying biological or physical manifestations.

For purposes of the present invention, an effective dose will be from about 0.01 mg/ kg to 50 mg/kg or 0.05 mg/kg to about 10 mg/kg of the polynucleotide, polypeptide or antibody compositions in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

### Delivery Methods

Once formulated, the pharmaceutical compositions of the invention can be (1) administered directly to the subject; (2) delivered *ex vivo,* to cells derived from the subject; or (3) delivered in vitro for expression of recombinant proteins.

Direct delivery of the compositions will generally be accomplished by injection, either subcutaneously, intraperitoneally, intravenously or intramuscularly, or delivered to the interstitial space of a tissue. The compositions can also be administered into a plaque or lesion. Other modes of administration include topical, oral, catheterized and pulmonary administration, suppositories, and transdermal applications, needles, and particle guns or hyposprays. Dosage treatment may be a single dose schedule or a multiple dose schedule.

Methods for the *ex vivo* delivery and reimplantation of transformed cells into a subject are known in the art and described in e.g., International Publication No. WO 93/14778. Examples of cells useful in *ex vivo* applications include, for example, stem cells, particularly hematopoetic, lymph cells, macrophages, dendritic cells, or tumor cells.

Generally, delivery of nucleic acids for both *ex vivo* and *in vitro* applications can be accomplished by, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Various methods are used to administer the therapeutic composition directly to a specific site in the body. For example, a small metastatic lesion is located and the therapeutic composition injected several times in several different locations within the body of tumor. Alternatively, arteries which serve a tumor are identified, and the therapeutic composition injected into such an artery, in order to deliver the composition directly into the tumor. A tumor that has a necrotic center is aspirated and the composition injected directly into the now empty center of the tumor. The antisense composition is directly administered to the surface of the tumor, for example, by topical application of the composition. X-ray imaging is used to assist in certain of the above delivery methods.

Receptor-mediated targeted delivery of therapeutic compositions containing an antisense polynucleotide; subgenomic polynucleotides, or antibodies to specific tissues is also used. Receptor-mediated DNA delivery techniques are described in, for example, Findeis et al., Trends in Biotechnol. (1993) 11:202-205; Chiou et al., (1994) Gene Therapeutics: Methods And Applications Of Direct Gene Transfer (J. A. Wolff, ed.); Wu et al., J. Biol. Chem. (1994) 269:542-46. Preferably, receptor-mediated targeted delivery of therapeutic compositions containing antibodies of the invention is used to deliver the antibodies to specific tissue.

Pharmaceutical compositions containing antisense, ribozyme or RNAi polynucleotides are administered in a range of about 100 ng to about 200 mg of polynucleotides for local administration in a gene therapy protocol. Concentration ranges of about 500 ng to about 50 mg, about 1 µg to about 2 mg, about 5 µg to about 500 µg, and about 20 µg to about 100 µg of polynucleotides can also be used during a gene therapy protocol. Factors such as method of action and efficacy of transformation and expression are considerations which will affect the dosage required for ultimate efficacy of the polynucleotides. Where greater expression is desired over a larger area of tissue, larger amounts of polynucleotides or the same amounts readministered in a successive protocol of administrations, or several administrations to different adjacent or close tissue portions of, for example, a tumor site, may be required to effect a positive therapeutic outcome. In all cases, routine experimentation in clinical trials will determine specific ranges for optimal therapeutic effect. A more complete description of gene therapy vectors, especially retroviral vectors, is contained in U.S. Ser. No. 08/869,309, which is expressly incorporated herein.

All reference referred to herein are incorporated by reference herein in their entirety.

The present invention will now be illustrated by way of the following non limiting examples.

### EXAMPLES

Example 1. Extraction, purification and digestion.

Plaques were grinded in liquid nitrogen and approximately 500 ul of powder/plaque was used for extraction. Proteins were extracted using 500 ul 40 mM Tris buffer pH 7.5 plus an 1x EDTA-free proteinase inhibitor cocktail (Roche) and 20 sec sonification on ice. After centrifugation (13000 rpm 4°C, 10 min), the sup was stored at -80°C as the Tris fraction. The remaining pellet was extracted with 1 ml of 75% Chloroform/25% Methanol with 20 sec sonification. After centrifugation (13000 rpm 4°C, 10 min), the sup was stored as the Lipid fraction and the pellet was extracted using 0.1% SDS. For this, 300 ul 0.1% SDS was added to the pellet followed by 20 sec sonification and heated for 5 min at 95°C. After centrifugation (13000 rpm 4°C, 10 min) the sup was used as the SDS fraction and stored at -80°C on ice. Both the Tris and the SDS fraction were used in the analysis.

First analysis was focused on the proteomics comparing patients that had a cardiovascular event and event-free controls. For this, plaque proteins of 79 patients were pooled that had an event within the 2 years of follow up and compared to a pool of plaque proteins of 79 sex and age matched patients that did not have an event in the same follow up time or longer.

For each group (event & control), 1.5 mg pooled protein of the Tris fraction was used with for every individual patient 19 ug. For each group, 0.5 mg pooled protein was used with 6.5 ug for every individual patient. To remove the 6 most abundant serum proteins (albumin, IgG, IgA, transferrin, haptoglobin, and antitrypsin), the Multiple Affinity Removal Spin Cartridge from Agilent (5188-5341) was used.

After purification, 400 ug of the purified Tris protein and 100 ug of purified SDS protein were used for digestion.

Before digestion the sample was split in two to get 2 technical replicates for each sample.

Samples were reduced, alkylated, and tryptic digested as described (Washburn, M. P. et al, (2001) Nat Biotechnol 19, 242-247). The samples were then desalted by passing the digested mixture through a conditioned Sep-Pak C-18 SPE cartridge (Waters, Milford, MA, USA), washed twice with a 3% acetonitrile (ACN) (JT Baker, Phillipsburg, NJ) and 0.1% formic acid (FA) buffer, and eluted with a 70% ACN and 0.1% FA buffer. The eluted samples were then dried to about 20 ul by removing organic solvent in a speedvac.

The samples were kept at 4°C and separated off-line on a BioBasic SCX column (5um, Thermo Electron, San Jose, USA) using an Agilent 1100 HPLC system. The Tris fractions were separated in 10 salt fractions (0, 2, 4, 6, 10, 18, 26, 45, 100, 1000 mM) loading 80 ul sample or salt and using a flow 10 ul/min (4% Acetonitril, 0.1% Formic Acid) for 30 min. The SDS fraction was separated in 5 fractions (0, 4, 18, 100, 1000 mM). Each salt-fraction was concentrated using a speedvac and injected separately onto the second dimension for MS analysis.

The second dimensional chromatographic separation was carried out with a home-packed nanobored C18 column (75um i.d x 10cm, 5µm particles) directly into a pico-frit nanospray tip (New Objective, Wubrun, MA, USA), operating at a flow rate of 200 nL/min with a 65 min gradient. The LTQ was operated in a data-dependent mode by performing MS/MS scans for the 3 of the most intense peaks from each MS scan. The MS was operated at an nanospray voltage of 1.8 kV; without shealth gas and auxiliary gas flow; ion transfer tube temperature of 180°C; collision gas pressure of 0.85 mTorr and normalized collision energy at 35% for MS2. Ion selection threshold was set to 500 counts for initiating MS2 while activation q was set to 0.25 and activation time to 30 ms. The MS scan range was 400-1800 m/z. Dynamic exclusion was activated with an exclusion duration of 1 min. For each salt step experiment, ms/ms (dta) spectra were extracted from the raw data files using the Biowork 3.3 program (ThermoFinnigan, San Jose, CA, USA).

Protein identification was achieved by searching the data from each salt step against the IPI human protein database (version 3.15, 58009 entries; Kersey P. J., Duarte J., Williams A., Karavidopoulou Y., Birney E., Apweiler R. The International Protein Index: An integrated database for proteomics experiments. Proteomics 4(7): 1985-1988 (2004)) using Sequest (Bioworks 3.3) allowing a maximum of 3 missed cleavages using trypsin. Fixed modification was carbaminomethylation and variable modification was oxidation of methionine. Results filters used: for peptides XCorr set for z=1 at 1.5, z=2 at 2.5 and z=3 at 3.5 and for proteins protein probability set at 1.00E-3.

### Bioinformatics

Using Bioworks, one Excel™ file with identified proteins was generated per salt fraction run resulting for the TRIS fraction in 2 x 10 Excel files for Events and 2 x 10 Excel™ files for the matched controls. For the SDS fraction 2 x 5 Excel™ files with identified proteins were generated for events as well as for the matched controls.

Omniviz™ and Excel™ were used to select for proteins that were only present in both technical replicas of the events ( at least in 1 salt-fraction) and not present in either technical replica (in none of the salt-fractions) of the matched controls.

This resulted in a list of 112 proteins that were submitted to Ingenuity software for further analysis. Ingenuity recognized 87 of the 112 proteins that could be used for building networks (see table I).

**Table I List of 87 human plaque proteins that differ between patients that had a major cardiovascular events within 2 years after the removal of the carotid plaque (carotid atherectomy) and sex and age matched controls and that were selected by Ingenuity for building networks.**

| IPI | name | Description |
|---|---|---|
| IP100647650 | EIF3S3 | eukaryotic translation initiation factor 3, subunit 3 gamma, 40kDa |
| IPI00647400 | RNPEP | arginyl aminopeptidase (aminopeptidase B) |
| IPI00555610 | AHNAK | AHNAK nucleoprotein (desmoyokin) |
| IPI00550917 | TWF2 | twinfilin, actin-binding protein, homolog 2 (Drosophila) |
| IPI00472175 | TXNRD1 | thioredoxin reductase 1 |
| IPI00418169 | ANXA2 | annexin A2 |
| IPI00413728 | SPTAN1 | spectrin, alpha, non-erythrocytic 1 (alpha-fodrin) |
| IPI00386854 | HNRPA2B1 | heterogeneous nuclear ribonucleoprotein A2/B1 |
| IPI00384051 | PSME2 | proteasome (prosome, macropain) activator subunit 2 (PA28 beta) |
| IPI00382470 | HSP90AA1 | heat shock protein 90kDa alpha (cytosolic), class A member 1 |
| IPI00301579 | NPC2 | Niemann-Pick disease, type C2 |
| IPI00299738 | PCOLCE | procollagen C-endopeptidase enhancer |
| IPI00291006 | MDH2 | malate dehydrogenase 2, NAD (mitochondrial) |
| IPI00290462 | CBR3 | carbonyl reductase 3 |
| IPI00289758 | CAPN2 | calpain 2, (m/II) large subunit |
| IPI00258514 | HSPA5 | heat shock 70kDa protein 5 (glucose-regulated protein, 78kDa) |
| IPI00221255 | MYLK | myosin, light chain kinase |
| IPI00220857 | CAST | Calpastatin |
| IPI00220741 | SPTA1 | spectrin, alpha, erythrocytic 1 (elliptocytosis 2) |
| IPI00220327 | KRT1 | keratin 1 (epidermolytic hyperkeratosis) |
| IPI00220271 | AKR1A1 | aldo-keto reductase family 1, member A1 (aldehyde reductase) |
| IPI00219525 | PGD | phosphogluconate dehydrogenase |
| IPI00218782 | CAPZB | capping protein (actin filament) muscle Z-line, beta |
| IPI00218746 | C1QB | complement component 1, q subcomponent, B chain |
| IPI00218446 | ADH5 | alcohol dehydrogenase 5 (class III), chi polypeptide integrin, beta 1 (fibronectin receptor, beta polypeptide, antigen CD29 includes MDF2, |
| IPI00217561 | ITGB1 | MSK12) |
| IPI00217236 | TBCA | tubulin folding cofactor A |
| IPI00216592 | HNRPC | heterogeneous nuclear ribonucleoprotein C (C1/C2) |
| IPI00216513 | SPTB | spectrin, beta, erythrocytic (includes spherocytosis, clinical type I) |
| IPI00215746 | FABP4 | fatty acid binding protein 4, adipocyte |
| IPI00168728 | IGHG1 | immunoglobulin heavy constant gamma 1 (G1m marker) |
| IPI00165421 | SERPINC1 | serpin peptidase inhibitor, clade C (antithrombin), member 1 |
| IPI00163187 | FSCN1 | fascin homolog 1, actin-bundling protein (Strongylocentrotus purpuratus) |
| IPI00154515 | SNIP1 | Smad nuclear interacting protein 1 |
| IPI00106668 | M6PRBP1 | mannose-6-phosphate receptor binding protein 1 |
| IPI00073772 | FBP1 | fructose-1,6-bisphosphatase 1 |
| IPI00056357 | C19ORF10 | chromosome 19 open reading frame 10 |
| IPI00033494 | MRLC2 | myosin regulatory light chain MRLC2 |
| IPI00031091 | EFHD1 | EF-hand domain family, member D1 |
| IPI00031008 | TNC | tenascin C (hexabrachion) |
| IPI00030275 | TRAP1 | TNF receptor-associated protein 1 |
| IPI00029111 | DPYSL3 | dihydropyrimidinase-like 3 heterogeneous nuclear ribonucleoprotein D (AU-rich element RNA binding protein 1, |
| IPI00028888 | HNRPD | 37kDa) |
| IPI00027482 | SERPINA6 | serpin peptidase inhibitor, clade A (alpha-1 antiproteinase, antitrypsin), member 6 aggrecan 1 (chondroitin sulfate proteoglycan 1, large aggregating proteoglycan, |
| IPI00027377 | AGC1 | antigen identified by monoclonal antibody A0122) |
| IPI00026182 | CAPZA2 | capping protein (actin filament) muscle Z-line, alpha 2 |
| IPI00026156 | HCLS1 | hematopoietic cell-specific Lyn substrate 1 |
| IPI00024107 | SNCA | synuclein, alpha (non A4 component of amyloid precursor) |
| IPI00024067 | CLTC | clathrin, heavy chain (Hc) |
| IPI00023601 | HAPLN1 | hyaluronan and proteoglycan link protein 1 |
| IPI00022394 | C1QC | complement component 1, q subcomponent, C chain |
| IPI00021405 | LMNA | lamin A/C secreted phosphoprotein 1 (osteopontin, bone sialoprotein I, early T-lymphocyte |
| IPI00021000 | SPP1 | activation 1) |
| IPI00020996 | IGFALS | insulin-like growth factor binding protein, acid labile subunit |
| IPI00020008 | NEDD8 | neural precursor cell expressed, developmentally down-regulated 8 |
| IPI00019579 | CFD | complement factor D (adipsin) |
| IPI00018769 | THBS2 | thrombospondin 2 |
| IPI00018352 | UCHL1 | ubiquitin carboxyl-terminal esterase L1 (ubiquitin thiolesterase) |
| IPI00018342 | AK1 | adenylate kinase 1 |
| IPI00018146 | YWHAQ | tyrosine 3-monooxygenase/tryptophan 5-monooxygenase activation protein, theta |
| | | polypeptide |
| IPI00017672 | NP | nucleoside phosphorylase |
| IPI00016915 | IGFBP7 | insulin-like growth factor binding protein 7 |
| IPI00016832 | PSMA1 | proteasome (prosome, macropain) subunit, alpha type, 1 |
| IPI00015842 | RCN1 | reticulocalbin 1, EF-hand calcium binding domain |
| | CALU (includes | |
| IPI00014537 | EG:813) | Calumenin |
| IPI00014424 | EEF1A2 | eukaryotic translation elongation factor 1 alpha 2 |
| IPI00013894 | STIP1 | stress-induced-phosphoprotein 1 (Hsp70/Hsp90-organizing protein) |
| IPI00013272 | GOLGA4 | golgi autoantigen, golgin subfamily a, 4 |
| IPI00013122 | CDC37 | cell division cycle 37 homolog (S. cerevisiae) |
| IPI00010860 | PSMD9 | proteasome (prosome, macropain) 26S subunit, non-ATPase, 9 |
| | | ras-related C3 botulinum toxin substrate 1 (rho family, small GTP binding protein |
| IPI00010271 | RAC1 | Rac1) |
| IPI00010257 | ERAF | erythroid associated factor |
| IPI00009920 | C6 | complement component 6 |
| IPI00009032 | SSB | Sjogren syndrome antigen B (autoantigen La) |
| IPI00009028 | CLEC3B | C-type lectin domain family 3, member B |
| IPI00008453 | CORO1C | coronin, actin binding protein, 1C |
| IPI00008433 | RPS5 | ribosomal protein S5 |
| IPI00007983 | PDLIM2 | PDZ and LIM domain 2 (mystique) |
| IPI00007047 | S100A8 | S100 calcium binding protein A8 |
| IPI00006725 | DDX23 | DEAD (Asp-Glu-Ala-Asp) box polypeptide 23 |
| | | serpin peptidase inhibitor, clade F (alpha-2 antiplasmin, pigment epithelium derived |
| IPI00006114 | SERPINF1 | factor), member 1 |
| | CAPZA1 (includes | |
| IPI00005969 | EG:829) | capping protein (actin filament) muscle Z-line, alpha 1 |
| IPI00005159 | ACTR2 | ARP2 actin-related protein 2 homolog (yeast) |
| IPI00004503 | LAMP1 | lysosomal-associated membrane protein 1 |
| IPI00002459 | ANXA6 | annexin A6 |
| IPI00000792 | CRYZ | crystallin, zeta (quinone reductase) |
| IPI00000760 | DDAH2 | dimethylarginine dimethylaminohydrolase 2 |

To select proteins for validation in the individual patients, proteins were selected that were present in the Ingenuity networks that could be linked. Ingenuity analysis identified 8 networks (see Fig 1). Six networks (network 1-6) had a high score ranging from 18 up to 38. Moreover, all these 6 networks could be linked into 1 major network showing that the 82 selected differential expressed proteins for this major network are described to interact with each other and that they have a biological relationship. This network is presented as an Ingenuity network in Fig 2 showing direct and indirect relationships between the proteins and in Fig 3, the network is arranged according to localization in the cell. The carotid plaque proteins listed in the network have a very high potential to be predictive for the occurrence of future major cardiovascular events elsewhere in the body and these proteins are listed in table II. The complete list of proteins in the network, which would be suitable as marker according to the invention can be found in Table III.

**Table II List of 82 human plaque proteins that differ between patients that had a major cardiovascular events within 2 years after the removal of the carotid plaque (carotid atherectomy) and sex and age matched controls and that were selected by Ingenuity for building networks and were present in 1 major network that consists of 6 smaller networks.**

| International Protein Index | Name | Description |
|---|---|---|
| IPI00218446 | ADH5 | alcohol dehydrogenase 5 (class III), chi polypeptide |
| IPI00018342 | AK1 | adenylate kinase 1 |
| IPI00418169 | ANXA2 | annexin A2 |
| IPI00002459 | ANXA6 | annexin A6 |
| IPI00215746 | FABP4 | fatty acid binding protein 4, adipocyte |
| | | heterogeneous nuclear ribonucleoprotein D (AU-rich element RNA binding protein |
| IPI00028888 | HNRPD | 1, 37kDa) |
| | | integrin, beta 1 (fibronectin receptor, beta polypeptide, antigen CD29 includes |
| IPI00217561 | ITGB1 | MDF2, MSK12) |
| IPI00021405 | LMNA | lam in A/C |
| | | ras-related C3 botulinum toxin substrate 1 (rho family, small GTP binding protein |
| IPI00010271 | RAC1 | Rac1) |
| IPI00165421 | SERPINC1 | serpin peptidase inhibitor, clade C (antithrombin), member 1 |
| | | serpin peptidase inhibitor, clade F (alpha-2 antiplasmin, pigment epithelium derived |
| IPI00006114 | SERPINF1 | factor), member 1 |
| | | secreted phosphoprotein 1 (osteopontin, bone sialoprotein I, early T-lymphocyte |
| IPI00021000 | SPP1 | activation 1) |
| IPI00216513 | SPTB | spectrin, beta, erythrocytic (includes spherocytosis, clinical type I) |
| IPI00018769 | THBS2 | thrombospondin 2 |
| IPI00472175 | TXNRD1 | thioredoxin reductase 1 |
| IPI00018352 | UCHL1 | ubiquitin carboxyl-terminal esterase L1 (ubiquitin thiolesterase) |
| | | aggrecan 1 (chondroitin sulfate proteoglycan 1, large aggregating proteoglycan, |
| IPI00027377 | AGC1 | antigen identified by monoclonal antibody A0122) |
| IPI00555610 | AHNAK | AHNAK nucleoprotein (desmoyokin) |
| IPI00289758 | CAPN2 | calpain 2, (m/II) large subunit |
| IPI00220857 | CAST | calpastatin |
| IPI00019579 | CFD | complement factor D (adipsin) |
| IPI00009028 | CLEC3B | C-type lectin domain family 3, member B |
| IPI00023601 | HAPLN1 | hyaluronan and proteoglycan link protein 1 |
| IPI00026156 | HCLS1 | hematopoietic cell-specific Lyn substrate 1 |
| IPI00216592 | HNRPC | heterogeneous nuclear ribohucleoprotein C (C1/C2) |
| IPI00020996 | IGFALS | insulin-like growth factor binding protein, acid labile subunit |
| IPI00168728 | IGHG1 | immunoglobulin heavy constant gamma 1 (G1m marker) |
| IPI00220327 | KRT1 | keratin 1 (epidermolytic hyperkeratosis) |
| IPI00004503 | LAMP1 | lysosomal-associated membrane protein 1 |
| IPI00221255 | MYLK | myosin, light chain kinase |
| IPI00301579 | NPC2 | Niemann-Pick disease, type C2 |
| IPI00299738 | PCOLCE | procollagen C-endopeptidase enhancer |
| IPI00007983 | PDLIM2 | PDZ and LIM domain 2 (mystique) |
| IPI00384051 | PSME2 | proteasome (prosome, macropain) activator subunit 2 (PA28 beta) |
| IPI00007047 | S100A8 | S100 calcium binding protein A8 |
| IPI00024107 | SNCA | synuclein, alpha (non A4 component of amyloid precursor) |
| IPI00220741 | SPTA1 | spectrin, alpha, erythrocytic 1 (elliptocytosis 2) |
| IPI00009032 | SSB | Sjogren syndrome antigen B (autoantigen La) |
| IPI00031008 | TNC | tenascin C (hexabrachion) |
| IPI00005159 | ACTR2 | ARP2 actin-related protein 2 homolog (yeast) |
| IPI00220271 | AKR1A1 | aldo-keto reductase family 1, member A1 (aldehyde reductase) |
| IPI00056357 | C19ORF10 | chromosome 19 open reading frame 10 |
| IPI00218746 | C1QB | complement component 1, q subcomponent, B chain |
| IPI00022394 | C1QC | complement component 1, q subcomponent, C chain |
| IPI00009920 | C6 | complement component 6 |
| | CALU (includes | |
| IPI00014537 | EG:813) | calumenin |
| | CAPZA1 (includes | |
| IPI00005969 | EG:829) | capping protein (actin filament) muscle Z-line, alpha 1 |
| IPI00026182 | CAPZA2 | capping protein (actin filament) muscle Z-line, alpha 2 |
| IPI00218782 | CAPZB | capping protein (actin filament) muscle Z-line, beta |
| IPI00290462 | CBR3 | carbonyl reductase 3 |
| IPI00013122 | CDC37 | cell division cycle 37 homolog (S. cerevisiae) |
| IPI00024067 | CLTC | clathrin, heavy chain (Hc) |
| IPI00008453 | CORO1C | coronin, actin binding protein, 1C |
| IPI00000792 | CRYZ | crystallin, zeta (quinone reductase) |
| IPI00014424 | EEF1A2 | eukaryotic translation elongation factor 1 alpha 2 |
| IPI00647650 | EIF3S3 | eukaryotic translation initiation factor 3, subunit 3 gamma, 40kDa |
| IPI00010257 | ERAF | erythroid associated factor |
| IPI00073772 | FBP1 | fructose-1,6-bisphosphatase 1 |
| IPI00163187 | FSCN1 | fascin homolog 1, actin-bundling protein (Strongylocentrotus purpuratus) |
| IPI00013272 | GOLGA4 | golgi autoantigen, golgin subfamily a, 4 |
| IPI00386854 | HNRPA2B1 | heterogeneous nuclear ribonucleoprotein A2/B1 |
| IPI00382470 | HSP90AA1 | heat shock protein 90kDa alpha (cytosolic), class A member 1 |
| IPI00258514 | HSPA5 | heat shock 70kDa protein 5 (glucose-regulated protein, 78kDa) |
| IPI00016915 | IGFBP7 | insulin-like growth factor binding protein 7 |
| IPI00106668 | M6PRBP1 | mannose-6-phosphate receptor binding protein 1 |
| IPI00291006 | MDH2 | malate dehydrogenase 2, NAD (mitochondrial) |
| IPI00033494 | MRLC2 | myosin regulatory light chain MRLC2 |
| IPI00020008 | NEDD8 | neural precursor cell expressed, developmentally down-regulated 8 |
| IPI00017672 | NP | nucleoside phosphorylase |
| IPI00016832 | PSMA1 | proteasome (prosome, macropain) subunit, alpha type, 1 |
| IPI00010860 | PSMD9 | proteasome (prosome, macropain) 26S subunit, non-ATPase, 9 |
| IPI00015842 | RCN1 | reticulocalbin 1, EF-hand calcium binding domain |
| IPI00008433 | RPS5 | ribosomal protein S5 |
| IPI00154515 | SNIP1 | Smad nuclear interacting protein 1 |
| IPI00413728 | SPTAN1 | spectrin, alpha, non-erythrocytic 1 (alpha-fodrin) |
| IPI00013894 | STIP1 | stress-induced-phosphoprotein 1 (Hsp70/Hsp90-organizing protein) |
| IPI00217236 | TBCA | tubulin folding cofactor A |
| IPI00006725 | DDX23 | DEAD (Asp-Glu-Ala-Asp) box polypeptide 23 |
| IPI00029111 | DPYSL3 | dihydropyrimidinase-like 3 |
| IPI00219525 | PGD | phosphogluconate dehydrogenase |
| IPI00030275 | TRAP1 | TNF receptor-associated protein 1 |
| IPI00018146 | YWHAQ | tyrosine 3-monooxygenase/tryptophan 5-monooxygenase activation protein, theta polypeptide |

**Table III. List of all proteins occurring in the major Ingenuity network. Under log ratio is indicated whether the protein is overexpressed/present in the plaque (value 2) or underexpressed/absent in the plaque (value -2).**

| Name | Synonyms | Description | International Protein Index | Entrez Gene ID for Human | Log Ratio |
|---|---|---|---|---|---|
| | ABC37, AI414027, AI449286, | ATP-binding cassette, sub- | | | |
| | CMD1O, FLJ36852, SUR2, | family C (CFTR/MRP), member | | | |
| ABCC9 | SUR2A, SUR2B | 9 | | 10060 | |
| | 4921510D23Rik, AA409782, Actin- | | | | |
| | like protein 2, ARP2, D6Ertd746e, | ARP2 actin-related protein 2 | | | |
| ACTR2 | MGC36604, MGC95085 | homolog (yeast) | IPI00005159 | 10097 | 2.0 |
| | ADH-3, ADHX, CLASS III ADH, | alcohol dehydrogenase 5 (class | | | |
| ADH5 | FDH, Gs fdh, GSNOR | III), chi polypeptide | IPI00218446 | 128 | 2.0 |
| | Agc, AGC1, AGCAN, AGGRECAN, | | | | |
| | AGGRECAN G3 SPLICE | | | | |
| | VARIANT, cmd, Cspcp, CSPG1, | | | | |
| AGC1 | CSPGCP, MSK16, SEDK | aggrecan | IPI00027377 | 176 | 2.0 |
| | AGRT, ANTI-SATIETY PEPTIDE, | agouti related protein homolog | | | |
| AGRP | ART, ASIP2, Marp, MGC118963 | (mouse) | | 181 | |
| | 1110004P15RIK, 2310047C17RIK, | | | | |
| | AA589382, AHNAK 1, AHNAKRS, | | | | |
| | AV091586, DA, desmoyokin, DY6, | | | | |
| | Hypothetical PDZ Domain Protein, | | | | |
| AHNAK | MGC5395 | AHNAK nucleoprotein | IPI00555610 | 79026 | 2.0 |
| AK1 | B430205N08Rik, MGC108678 | adenylate kinase 1 | IPI00018342 | 203 | 2.0 |
| | 2610201A18Rik, Akr1a4, Akra, | aldo-keto reductase family 1, | | | |
| | ALDEHYDE REDUCTASE, ALDR1, | member A1 (aldehyde | | | |
| AKR1A1 | ALR, MGC12529, MGC1380 | reductase) | IPI00220271 | 10327 | 2.0 |
| | 12-lipoxygenase, 12-LO, 12-LOX, | | | | |
| | 12/15-Lox, 12S LIPOXYGENASE, | | | | |
| | 9930022G08Rik, Alox12p, | | | | |
| | Alox12_predicted, LOG12, P(12s)- | | | | |
| | lox, P-12LO, PLATELET TYPE 12- | | | | |
| ALOX12 | LO | arachidonate 12-lipoxygenase | | 239 | |
| | ANNEXIN II, ANNEXIN2, ANX2, | | | | |
| | ANX2L4, AW215814, CAL1H, | | | | |
| | Calpactin-1 heavy chain, LIP2, | | | | |
| | LIPOCORTIN2, LPC2, LPC2D, | | | | |
| ANXA2 | P36, PAP-IV | annexin A2 | IPI00418169 | 302 | 2.0 |
| | Annexin VI, ANNEXIN6, ANX6, | | | | |
| | AnxVl, AW107198, Cabm, | | | | |
| | CALPHOBINDIN2, Camb, CBP68, | | | | |
| ANXA6 | CPB-II, p68 | annexin A6 | IPI00002459 | 309 | 2.0 |
| | 2210008M02Rik, AI647473, AT3, | | | | |
| | ATAXIN 3, ATX3, JOS, MJD, | | | | |
| ATXN3 | MJD1, Rsca3, SCA3 | ataxin 3 | | 4287 | |
| | ASP2, BACE, BETA SECRETASE, | | | | |
| | C76936, FLJ90568, HSPC104, | beta-site APP-cleaving enzyme | | | |
| BACE1 | KIAA1149, MEMAPSIN 2 | 1 | | 23621 | |
| | AI256696, AW045498, BRCC2, | | | | |
| | FACD, FAD, FAD1, FANCB, | | | | |
| BRCA2 | FANCD, FANCD1, RAB163 | breast cancer 2, early onset | | 675 | |
| | D17Wsu104e, | | | | |
| | EUROIMAGE1875335, IL25, IL27, | | | | |
| | IL27w, Interleukin-25, Ly6elg, | chromosome 19 open reading | | | |
| C19ORF10 | R33729 1, SF20 | frame 10 | IPI00056357 | 56005 | 2.0 |
| | | complement component 1, q | | | |
| C1QB | C1q Beta-Chain | subcomponent, B chain | IPI00218746 | 713 | 2.0 |
| C1QC | AI385742, C1QG, Ciqc, FLJ27103, | complement component 1, q | IPI00022394 | 714 | 2.0 |
| | MGC105681 | subcomponent, C chain | | | |
| C6 | AW111623 | complement component 6 | IPI00009920 | 729 | 2.0 |
| C9 | | complement component 9 | | 735 | |
| CALU | 9530075H20RIK, Calu, | | | | |
| (includes | CALUMENIN, FLJ90608, | | | | |
| EG:813) | Loc360380, MGC124555 | calumenin | IPI00014537 | 813 | 2.0 |
| | AI326419, CALCIUM ACTIVATED | | | | |
| | NEUTRAL PROTEINASE, | | | | |
| | CALPAIN II, Calpain2, CANPL2, | | | | |
| | CANPml, Capa-2, FLJ39928, M | | | | |
| | CALPAIN, M-CALPIN, M80, | | | | |
| CAPN2 | mCANP | calpain 2, (m/II) large subunit | IPI00289758 | 824 | 2.0 |
| CAPZA1 | | | | | |
| (includes | | capping protein (actin filament) | | | |
| EG:829) | CAPPA1, CAPZ, CAZ1 | muscle Z-line, alpha 1 | IPI00005969 | 829 | 2.0 |
| | 1110053K06RIK, AW208808, | | | | |
| | CAPPA2, Capping Protein alpha2, | capping protein (actin filament) | | | |
| CAPZA2 | CAPZ, Capz Alpha2, MGC124519 | muscle Z-line, alpha 2 | IPI00026182 | 830 | 2.0 |
| | 1700120C01Rik, AI325129, CAPB, | | | | |
| | CAPPB, Cappb1, Capping protein | | | | |
| | beta 1, Capping protein beta 2, | | | | |
| | CAPZ, Capz beta, CPB1, CPB2, | | | | |
| | CPbeat2, CPbeta1, CPbeta2, F- | | | | |
| | Actin Capping Protein beta, F- | | | | |
| | CAPPING, MGC104401, | | | | |
| | MGC129749, MGC129750, | capping protein (actin filament) | | | |
| CAPZB | MGC95097 | muscle Z-line, beta | IPI00218782 | 832 | 2.0 |
| | BS-17, CALPASTATIN, CAST-831, | | | | |
| | HEART TYPE CALPASTATIN, | | | | |
| CAST | MGC108997, MGC9402 | calpastatin | IPI00220857 | 831 | 2.0 |
| | 1110001J05Rik, C81353, Carbonyl | | | | |
| | reductase-3, Cbr3_predicted, | | | | |
| CBR3 | hCBR3, MGC99956 | carbonyl reductase 3 | IPI00290462 | 874 | 2.0 |
| | C75951, LAMP-3, LIMP, ME491, | | | | |
| | MGC103180, MGC107286, | | | | |
| | MGC72893, MLA1, OMA81H, | | | | |
| CD63 | TSPAN30 | CD63 molecule | | 967 | |
| | 5H9, BA2, BTCC-1, CD9 | | | | |
| | ANTIGEN, DRAP-27, GIG2, MIC3, | | | | |
| CD9 | MRP-1, P24, TSPAN29 | CD9 molecule | | 928 | |
| | | cell division cycle 37 homolog | | | |
| CDC37 | P50, P50CDC37 | (S. cerevisiae) | IPI00013122 | 11140 | 2.0 |
| | C/EBP ALPHA, C/EBP ALPHA | | | | |
| | P30, C/EBP ALPHA P42, CBF-A, | | | | |
| | CEBP, Cebp Alpha, DBPCEP, p42, | CCAAT/enhancer binding | | | |
| CEBPA | Zinc Finger Homeobox 1b | protein (C/EBP), alpha | | 1050 | |
| | ADIPSIN, ADN, Complement | | | | |
| | Factor D, DF, EVE, FACTOR D, | | | | |
| CFD | PFD | complement factor D (adipsin) | IPI00019579 | 1675 | 2.0 |
| | Clec3b_predicted, | | | | |
| | DKFZp686H17246, | C-type lectin domain family 3, | | | |
| CLEC3B | TETRANECTIN, TN, TNA, TTN | member B | IPI00009028 | 7123 | 2.0 |
| | 3110065L21Rik, CHC, CHC17, | | | | |
| | Clathrin Hc, CLATHRIN HEAVY | | | | |
| | CHAIN, CLH-17, CLTCL2, Hc, | | | | |
| CLTC | KIAA0034, MGC92975, R74732 | clathrin, heavy chain (Hc) | IPI00024067 | 1213 | 2.0 |
| | alpha3(IV), Collagen Type IV | | | | |
| | alpha3, Goodpasture antigen, | collagen, type IV, alpha 3 | | | |
| COL4A3 | tumstatin, [a]3(IV) | (Goodpasture antigen) | | 1285 | |
| | AL022675, AW455561, AW548837, | | | | |
| | coronin-3, HCRNN4, MGC102522, | coronin, actin binding protein, | | | |
| CORO1C | RGD1564490_predicted | 1C | IPI00008453 | 23603 | 2.0 |
| | Crystallin zeta, DKFZp779E0834, | | | | |
| | FLJ41475, Loc362061, QR, Sez9, | crystallin, zeta (quinone | | | |
| CRYZ | ZETA CRYSTALLIN | reductase) | IPI00000792 | 1429 | 2.0 |
| | Csfgm, GM-CSF, MGC131935, | | | | |
| | MGC138897, MGC151255, | colony stimulating factor 2 | | | |
| CSF2 | MGC151257, MGI-IGM, NIF | (granulocyte-macrophage) | | 1437 | |
| | Armadillo, Beta-cat, BETA- | | | | |
| | CATENIN, CATENIN BETA, | catenin (cadherin-associated | | | |
| CTNNB1 | CATNB, CTNNB, FLJ25606, Mesc | protein), beta 1, 88kDa | | 1499 | |
| | 1300003D18Rik, 4932411N15Rik, | | | | |
| | AI327301, Cul2_predicted, | | | | |
| | CULLIN2, KIAA4106, MGC131970, | | | | |
| CUL2 | mKIAA4106 | cullin 2 | | 8453 | |
| | BB139920, CMK, crg-10, Humig, | chemokine (C-X-C motif) ligand | | | |
| CXCL9 | MGC105312, MIG, SCYB9 | 9 | | 4283 | |
| | 3110082M05RIK, 4921506D17Rik, | | | | |
| | Ddx23_predicted, MGC8416, | | | | |
| | prp28, PRPF28, U5-100K, U5- | DEAD (Asp-Glu-Ala-Asp) box | | | |
| DDX23 | 100KD | polypeptide 23 | IPI00006725 | 9416 | -2.0 |
| | CRMP-4, | | | | |
| | DIHYDROPYRIMIDINASE | | | | |
| | RELATED PROTEIN-3, DRP-3, | | | | |
| | TUC-4b, TUC4, ULIP, ULIP protein, | | | | |
| DPYSL3 | Ulip1 | dihydropyrimidinase-like 3 | IPI00029111 | 1809 | -2.0 |
| | Eef1a, EEF1AL, EF-1-alpha-2, | | | | |
| | EF1A, EF1alpha, HS1, Ps10, | | | | |
| | RATPS10, S1, Statin, STN, Stnl, | eukaryotic translation elongation | | | |
| EEF1A2 | wasted, wst | factor 1 alpha 2 | IPI00014424 | 1917 | 2.0 |
| | 1810016104Rik, 2700079K05Rik, | | | | |
| | AA409117, AA409446, C78575, | | | | |
| | EIF3-ALPHA, EIF3-P35, eIF3j, | | | | |
| | Eif3s1 (predicted), | eukaryotic translation initiation | | | |
| EIF3S1 | Eif3s1_predicted, MGC156679 | factor 3, subunit 1 alpha, 35kDa | | 8669 | |
| | 36kDa, D4Ertd632e, eIF3-beta, | | | | |
| EIF3S2 | EIF3-P36, eIF3i, Eif3s2, PRO2242, | | | | |
| (includes | RGD1560498, Tgf Beta Receptor | eukaryotic translation initiation | | | |
| EG:8668) | Binding Protein, Tif3, TRIP-1 | factor 3, subunit 2 beta, 36kDa | | 8668 | |
| | 1110008A16Rik, 40kD, | | | | |
| | 9430017H16Rik, eIF3 p40 subunit, | | | | |
| | EIF3-gamma, EIF3-P40, eIF3h, | eukaryotic translation initiation | | | |
| | MGC102958, MGC107627, | factor 3, subunit 3 gamma, | | | |
| EIF3S3 | MGC72941 | 40kDa | IPI00647650 | 8667 | 2.0 |
| | 44kDa, D0Jmb4, EIF3-DELTA, | | | | |
| | EIF3-P42, EIF3-P44, eIF3g, | | | | |
| | Eukaryotic translation initiation | | | | |
| | factor 3 (p42 subunit), p44, TU- | eukaryotic translation initiation | | | |
| EIF3S4 | 189B2 | factor 3, subunit 4 delta, 44kDa | | 8666 | |
| | 0610037M02Rik, AA409853, EIF3- | eukaryotic translation initiation | | | |
| | EPSILON, EIF3-P47, eIF3f, | factor 3, subunit 5 epsilon, | | | |
| EIF3S5 | Eif3s5_predicted, MGC90606 | 47kDa | | 8665 | |
| | 66/67kDa, AA407891, EIF3-P66, | | | | |
| | eIF3-zeta, eIF3d, MGC115774, | eukaryotic translation initiation | | | |
| | MGC126526, MGC17258, | factor 3, subunit 7 zeta, | | | |
| EIF3S7 | MGC93918, MOE1 | 66/67kDa | | 8664 | |
| | 110kDa, 3230401013RIK, EIF3- | | | | |
| | P110, EIF3C, MGC36637, | | | | |
| | NIPIL(A3), NipilA3, Translation | eukaryotic translation initiation | | | |
| EIF3S8 | initiator factor 3s8 | factor 3, subunit 8, 110kDa | | 8663 | |
| | AL033316, AL033334, AL033369, | | | | |
| | AW208965, D12Mgi40, D5Wsu45e, | | | | |
| | EIF3-ETA, EIF3-P110, EIF3-P116, | | | | |
| | Eif3.9, eIF3b, MGC104664, | | | | |
| | MGC112720, MGC131875, p116, | eukaryotic translation initiation | | | |
| EIF3S9 | PRT1 | factor 3, subunit 9 eta, 116kDa | | 8662 | |
| | | epidermal growth factor receptor | | | |
| EPS8 | AW261790, Eps8_predicted | pathway substrate 8 | | 2059 | |
| | AHSP, EDRF, Eraf_predicted, | | | | |
| | Erythroid differentiatiation factor, | | | | |
| ERAF | MGC130147 | erythroid associated factor | IPI00010257 | 51327 | 2.0 |
| | C-ERBB2, c-neu, EGFR2, ERBB2 | | | | |
| | RECEPTOR, HER-2, HER2/NEU, | v-erb-b2 erythroblastic leukemia | | | |
| | mKIAA3023, NEU, NGL, | viral oncogene homolog 2, | | | |
| | p185erbb2, p185HER2, P185NEU, | neuro/glioblastoma derived | | | |
| ERBB2 | TKR1 | oncogene homolog (avian) | | 2064 | |
| | BETA ESTROGEN RECEPTOR, | | | | |
| | ER-BETA, ERB, ERB2, ER[b], | | | | |
| | ESR-BETA, ESRB, ESTRB, | | | | |
| | ESTROGEN RECEPTOR BETA, | | | | |
| ESR2 | NR3A2 | estrogen receptor 2 (ER beta) | | 2100 | |
| | 1600027G01Rik, AI503996, CF11, | coagulation factor XI (plasma | | | |
| F11 | factor XI, FACTOR XIA, | thromboplastin antecedent) | | 2160 | |
| | FACTOR11, FXI, LRRGT00086, | | | | |
| | MGC141891, PLATELET FACTOR | | | | |
| | XI | | | | |
| | ALPHA-THROMBIN, Cf-2, | | | | |
| | Coagulation factor 2, DCP, factor | | | | |
| | IIa, FII, Meizothrombin Des- | | | | |
| | Fragment 1, PROTHROMBIN, PT, | | | | |
| F2 | THROMBIN | coagulation factor II (thrombin) | | 2147 | |
| | 422/AP2, A-fab, A-FABP, Adipocyte | | | | |
| | fatty acid binding protein, ALBP, | fatty acid binding protein 4, | | | |
| FABP4 | ALBP/AP2, AP2, Ap4, LbpI | adipocyte | IPI00215746 | 2167 | 2.0 |
| FBLN2 | 5730577E14RIK, FIBULIN 2 | fibulin 2 | | 2199 | |
| | A55, ARMD3, DANCE, EVEC, | | | | |
| FBLN5 | FIBULIN 5, FLJ90059, UP50 | fibulin 5 | | 10516 | |
| | FBP, Fbp-2, FBPase 1, Fdp, | | | | |
| FBP1 | FRUCTOSE-BIPHOSPHATASE 1 | fructose-1,6-bisphosphatase 1 | IPI00073772 | 2203 | 2.0 |
| FGF18 | D130055P09RIK, ZFGF5 | fibroblast growth factor 18 | | 8817 | |
| | A1325264, C-FOS-INDUCED | | | | |
| | GROWTH FACTOR, VASCULAR | c-fos induced growth factor | | | |
| | ENDOTHELIAL GROWTH | (vascular endothelial growth | | | |
| FIGF | FACTOR D, VEGF-D | factor D) | | 2277 | |
| | | v-fos FBJ murine osteosarcoma | | | |
| FOS | C-FOS, D12Rfj1, V-FOS | viral oncogene homolog | | 2353 | |
| | Fkh14, FKHL14, Hfhbf3, LD, MFH- | forkhead box C2 (MFH-1, | | | |
| FOXC2 | 1, MFH-1 protein | mesenchyme forkhead 1) | | 2303 | |
| | | fascin homolog 1, actin-bundling | | | |
| | AI663989, Fan1, fascin, FASCIN1, | protein (Strongylocentrotus | | | |
| FSCN1 | FLJ38511, HSN, p55, SNL | purpuratus) | IPI00163187 | 6624 | 2.0 |
| | G-GLY, G17-GLY, GAS, GASTRIN, | | | | |
| GAST | Gastrin-17, PPG34 | gastrin | | 2520 | |
| | AI225887, AU019508, GCP2, | | | | |
| | GOLG, Golgin-245, LOC501069, | | | | |
| | MU-RMS-40.18, Olp-1, p230, | | | | |
| | SE20-7, Tgn p230, TGN230, Trans- | golgi autoantigen, golgin | | | |
| GOLGA4 | Golgi p230 | subfamily a, 4 | IPI00013272 | 2803 | 2.0 |
| | BB099155, CRTL1, CrtI1I, LINK, | hyaluronan and proteoglycan | | | |
| HAPLN1 | LINK PROTEIN, LP, MGC156657 | link protein 1 | IPI00023601 | 1404 | 2.0 |
| | AW213261, CTTNL, Hematopoietic | | | | |
| | specific protein 1, HS1, LCKBP1, | hematopoietic cell-specific Lyn | | | |
| HCLS1 | LYN SUBSTRATE 1 | substrate 1 | IPI00026156 | 3059 | 2.0 |
| | 2610109B09Rik, A930014B11Rik, | | | | |
| | E130315I21Rik, Hippi, ILWEQ, | | | | |
| | KIAA4113, MGC126506, | | | | |
| HIP1 | MGC27616, mKIAA4113 | huntingtin interacting protein 1 | | 3092 | |
| | 9130414A06Rik, hnRNP A, | | | | |
| | HNRNP A2, HNRNP B1, | | | | |
| | HNRNPA2/B1, hnRNPa2b1, | | | | |
| | HNRPA2, Hnrpa2b1 (predicted), | | | | |
| | Hnrpa2b1_predicted, HNRPB1, | heterogeneous nuclear | | | |
| HNRPA2B1 | RNPA2, SNRPB1, VDRE-BP2 | ribonucleoprotein A2/B1 | IPI00386854 | 3181 | 2.0 |
| | AL022939, C1, C2, D14Wsu171e, | | | | |
| | Heterogeneous nuclear | | | | |
| | ribonucleoprotein C, HNRNP, | | | | |
| | Hnrnp C1/C2, HNRNPC, | | | | |
| | HNRNPC1, HNRNPC2, Hnrpc1, | | | | |
| | Hnrpc2, MGC104306, | | | | |
| | MGC105117, MGC114359, | | | | |
| | MGC117353, MGC131677, | heterogeneous nuclear | | | |
| HNRPC | SNRPC | ribonucleoprotein C (C1/C2) | IPI00216592 | 3183 | 2.0 |
| | | heterogeneous nuclear | | | |
| | AUF1, AUF1A, C230004L04, | ribonucleoprotein D (AU-rich | | | |
| | HNRNP D, HNRNP D0, HnRNP | element RNA binding protein 1, | | | |
| HNRPD | UP2, P37 | 37kDa) | IPI00028888 | 3184 | 2.0 |
| | c-bas/has, C-H-RAS, C-HA-RAS, | | | | |
| | C-HA-RAS-1, c-rasHa, HA-RAS, | | | | |
| | Harvey-ras, HRAS1, K-ras, Kras2, | | | | |
| | N-ras, RAS, RAS HA, RASH1, V-H- | v-Ha-ras Harvey rat sarcoma | | | |
| HRAS | RAS | viral oncogene homolog | | 3265 | |
| | 86kDa, 89kDa, AL024080, | | | | |
| | AL024147, FLJ31884, Heat shock | heat shock protein 90kDa alpha | | | |
| HSP90AA1 | protein 1, alpha, HSP4, HSP86, | (cytosolic), class A member 1 | IPI00382470 | 3320 | 2.0 |
| | Hsp86-1, HSP89, Hsp90, HSP90 | | | | |
| | ALPHA, HSP90A, HSP90N, | | | | |
| | HSPC1, HSPCA, HSPCAL1, | | | | |
| | HSPCAL4, HSPN, LAP2, | | | | |
| | MGC105293 | | | | |
| | 78kDa, AL022860, AU019543, BIP, | | | | |
| | D2Wsu141e, D2Wsu17e, | | | | |
| | FLJ26106, GRP78, HEAT SHOCK | | | | |
| | 70KDA PROTEIN5, Hsce70, | | | | |
| | HSP70-5, Immunoglobulin heavy | heat shock 70kDa protein 5 | | | |
| | chain binding protein, mBiP, MIF2, | (glucose-regulated protein, | | | |
| HSPA5 | SEZ-7 | 78kDa) | IPI00258514 | 3309 | 2.0 |
| | 5-HT 2C RECEPTOR, 5-Ht | | | | |
| | receptor 2C, 5-HT2C, 5-HT2CR, 5- | 5-hydroxytryptamine (serotonin) | | | |
| HTR2C | HTR2C, 5HT1c, HTR1C, SR1 | receptor 2C | | 3358 | |
| | 4832416K17Rik, IDP ISOMERASE, | | | | |
| | Ipp delta isomerase, IPP | | | | |
| | ISOMERASE, IPP isomerase 1, | | | | |
| | IPP1, IPPI1, Isopentenyl | | | | |
| | diphosphate:dimethylallyl | | | | |
| | diphosphate isomerase, | | | | |
| | ISOPENTENYL | | | | |
| | PYROPHOSPHATE ISOMERASE, | | | | |
| | MGC108635, MGC118013, | | | | |
| | MGC8139, Similar to isopentenyl- | isopentenyl-diphosphate delta | | | |
| IDI1 | diphosphate delta isomerase | isomerase 1 | | 3422 | |
| | C730016P09Rik, des(1-3)IGF-I, | | | | |
| | Des-(1-3)-igf1, human | | | | |
| | somatomedin C, IGF-I, | | | | |
| | mecasermin, Preproinsulin-like | insulin-like growth factor 1 | | | |
| IGF1 | growth factor ia | (somatomedin C) | | 3479 | |
| | A330103N21RIK, CD221, | | | | |
| | D930020L01, hyft, IGF-IR, IGF1 | | | | |
| | RECEPTOR, Igf1r alpha, lgf1r beta, | | | | |
| | Igf1r beta isoform, IGFIRC, Igfr, | | | | |
| | IGFR1, JTK13, LOC51049, | | | | |
| | MGC142170, MGC142172, | insulin-like growth factor 1 | | | |
| IGF1R | MGC18216, NM1 | receptor | | 3480 | |
| | AI661837, CD222, Ci M6pr, Cl- | | | | |
| | MPR, lgf-II/mpr, lgfr2, M6P-R, | | | | |
| | M6P/IGF2R, MANNOSE-6- | | | | |
| | PHOSPHATE RECEPTOR, Mpr, | | | | |
| | MPR300, MPRI, TYPE 2 IGF | | | | |
| | RECEPTOR, Type 2 insulin-like | insulin-like growth factor 2 | | | |
| IGF2R | growth factor receptor | receptor | | 3482 | |
| | Albs, ALS, KIAA4111, | | | | |
| | MGC150140, MGC150141, | insulin-like growth factor binding | | | |
| IGFALS | mKIAA4111 | protein, acid labile subunit | IPI00020996 | 3483 | 2.0 |
| | AGM, FSTL2, IGFBP-7v, IGFBP- | | | | |
| | RP1, MAC25, PROSTACYCLIN | insulin-like growth factor binding | | | |
| IGFBP7 | STIMULATING FACTOR, PSF | protein 7 | IPI00016915 | 3490 | 2.0 |
| IGH@ | IGH, IGH.1@, IGHDY1 | immunoglobulin heavy locus | | 3492 | |
| | Gamma1, IG HEAVY CONSTANT | | | | |
| | GAMMA 1 (G1M MARKER), IGG | | | | |
| | GAMMA 1 HEAVY CHAIN, IGG | | | | |
| | KAPPA HEAVY CHAIN, IGG1, Igh- | | | | |
| | 4, IGH3, Ighg, Immunoglobulin GL | immunoglobulin heavy constant | | | |
| IGHG1 | Gamma 1, VH7183 | gamma 1 (G1m marker) | IPI00168728 | 3500 | 2.0 |
| | | immunoglobulin heavy constant | | | |
| IGHG2 | DKFZp686104196, IGG2 | gamma 2 (G2m marker) | | 3501 | |
| | DKFZp686H11213, FLJ39988, | | | | |
| | FLJ40036, FLJ40253, FLJ40587, | | | | |
| | FLJ40789, FLJ40834, IG HEAVY | | | | |
| | CONSTANT GAMMA3, IGG3, | | | | |
| | IGH3, Immunoglobulin Heavy | immunoglobulin heavy constant | | | |
| IGHG3 | Constant Gamma3, MGC45809 | gamma 3 (G3m marker) | | 3502 | |
| | | immunoglobulin heavy constant | | | |
| IGHG4 | MGC117419 | gamma 4 (G4m marker) | | 3503 | |
| | BSF1, IgG1, II4e12, INTERLEUKIN | | | | |
| IL4 | 4, MGC79402 | interleukin 4 | | 3565 | |
| | 4633401G24RIK, AA409975, | integrin, beta 1 (fibronectin | | | |
| ITGB1 | AA960159, BETA1 INTEGRIN, | receptor, beta polypeptide, | IPI00217561 | 3688 | 2.0 |
| | BETA1 INTEGRIN RECEPTOR, | antigen CD29 includes MDF2, | | | |
| | CD29, CD29 antigen, FNRB, | MSK12) | | | |
| | GPIIA, INTEGRIN BETA 1, | | | | |
| | INTEGRIN BETA 1 RECEPTOR, | | | | |
| | Integrin beta 1a, INTEGRIN BETA | | | | |
| | 1C, MDF2, MGC137337, MSK12, | | | | |
| | VLAB | | | | |
| | CK1, EHK1, K1, Kb1, KERATIN 1, | | | | |
| | KERATIN, 67K TYPE II | | | | |
| | CYTOSKELETAL, Krt-2.1, KRT1A, | keratin 1 (epidermolytic | | | |
| KRT1 | Krt2-1, Krt86 | hyperkeratosis) | IPI00220327 | 3848 | 2.0 |
| | AI196048, CD107A, LAMPA, | lysosomal-associated | | | |
| LAMP1 | Lgp110, LGP120 | membrane protein 1 | IPI00004503 | 3916 | 2.0 |
| | CDF, cholinergic differentiation | leukemia inhibitory factor | | | |
| | factor, D-FACTOR, HILDA, | (cholinergic differentiation | | | |
| LIF | LEUKEMIA INHIBITORY FACTOR | factor) | | 3976 | |
| | CD118, GP190, LEUKEMIA | | | | |
| | INHIBITORY FACTOR | | | | |
| | RECEPTOR, LIF, LIF RECEPTOR, | | | | |
| | LIFR ALPHA, LIFR BETA, SJS2, | leukemia inhibitory factor | | | |
| LIFR | STWS, SWS | receptor alpha | | 3977 | |
| | CDCD1, CDDC, CMD1A, CMT2B1, | | | | |
| | EMD2, FPL, FPLD, HGPS, IDC, | | | | |
| | LAMIN A, LAMIN A/C, Lamin C, | | | | |
| | Lamin C2, LAMININ A/C, LDP1, | | | | |
| | LFP, LGMD1B, Lmn, LMN1, LMNC, | | | | |
| LMNA | PRO1 | lamin A/C | IPI00021405 | 4000 | 2.0 |
| | 1300012C15Rik, MGC11117, | mannose-6-phosphate receptor | | | |
| M6PRBP1 | MGC2012, PP17, TIP47 | binding protein 1 | IPI00106668 | 10226 | 2.0 |
| | ABP620, ACF7, Aclp7, Actin cross- | | | | |
| | linking family protein 7, ACTIN- | | | | |
| | CROSSLINKING PROTEIN 7, | | | | |
| | FLJ45612, FLJ46776, KIAA0465, | | | | |
| MACF1 | KIAA1251, MACF, mACF7, | | | | |
| (includes | mKIAA0465, OFC4, R74989, | microtubule-actin crosslinking | | | |
| EG:23499) | TRABECULIN-ALPHA | factor 1 | | 23499 | |
| | COLEC1, HSMBPC, L-MBP, MBL, | | | | |
| | MBL-C, MBP, MBP-C, MBP1, | mannose-binding lectin (protein | | | |
| MBL2 | MGC116832, MGC116833 | C) 2, soluble (opsonic defect) | | 4153 | |
| | | malate dehydrogenase 2, NAD | | | |
| MDH2 | M-MDH, MDH, MGC:3559, MOR1 | (mitochondrial) | IPI00291006 | 4191 | 2.0 |
| | AV377895, SL-2, STMY2, | matrix metallopeptidase 10 | | | |
| MMP10 | Stromelysin 2 | (stromelysin 2) | | 4319 | |
| | | matrix metallopeptidase 11 | | | |
| MMP11 | SL-3, ST3, STMY3, Stromelysin 3 | (stromelysin 3) | | 4320 | |
| | 1500001M02Rik, C77744, | | | | |
| | MGC114341, MLC-B, MLC20, | | | | |
| | Mrlcb, Mylc2b, MYOSIN | | | | |
| | REGULATORY LIGHT CHAIN, | myosin regulatory light chain | | | |
| MRLC2 | RLC-B, Rmlc | MRLC2 | IPI00033494 | 103910 | 2.0 |
| | 5033418D15Rik, Beta-dg, | | | | |
| | FLJ31098, GCDP, MGC108675, | | | | |
| | MYOTROPHIN, Scid | | | | |
| MTPN | complementing gene 2, V-1 | myotrophin | | 136319 | |
| | 9530072E15RIK, A930019C19RIK, | | | | |
| | AW489456, DKFZp686110125, | | | | |
| | FLJ12216, KRP, MLCK, MLCK108, | | | | |
| | MLCK210, MSTP083, | | | | |
| MYLK | Mylk_predicted, Smmlck, TELOKIN | myosin, light chain kinase | IPI00221255 | 4638 | 2.0 |
| | NCF1, NOXO2, p47, | | | | |
| | p47<phox>, P47PHOX, | neutrophil cytosolic factor 1C | | | |
| NCF1C | PHOX47, SH3PXD1C | pseudogene | | 654817 | |
| | | neural precursor cell expressed, | | | |
| | MGC104393, MGC105320, | developmentally down-regulated | | | |
| NEDD8 | MGC125896, MGC125897, RUB1 | 8 | IPI00020008 | 4738 | 2.0 |
| | AL024301, AU015798, | | | | |
| | MGC117396, MGC125915, | | | | |
| | MGC125916, Np-1, Np-2, | | | | |
| | NUCLEOSIDE | | | | |
| | PHOSPHORYLASE, PNP, | | | | |
| | PRO1837, PUNP, PURINE | | | | |
| NP | PHOSPHORYLASE | nucleoside phosphorylase | IPI00017672 | 4860 | 2.0 |
| | 2700012J19Rik, AA408070, | | | | |
| | AU045843, EPIDIDYMAL | | | | |
| | SECRETORY PROTEIN, HE1, | | | | |
| NPC2 | MGC1333, re1 | Niemann-Pick disease, type C2 | IPI00301579 | 10577 | 2.0 |
| | CT-PCPE, MGC105446, PCPE, | | | | |
| | PCPE-1, Procollagen c-proteinase | procollagen C-endopeptidase | | | |
| PCOLCE | enhancer protein | enhancer | IPI00299738 | 5118 | 2.0 |
| | FLJ34715, MGC37634, | PDZ and LIM domain 2 | | | |
| PDLIM2 | MGC94060, mystique, SLIM | (mystique) | IPI00007983 | 64236 | 2.0 |
| | CD31, Er-Mp12, MGC102160, | platelet/endothelial cell adhesion | | | |
| PECAM1 | MGC124998, PECAM | molecule (CD31 antigen) | | 5175 | |
| | 0610042A05Rik, 6PGD, | phosphogluconate | | | |
| PGD | AU019875, C78335, Cc2-27 | dehydrogenase | IPI00219525 | 5226 | -2.0 |
| | CPK, Cpk-m, DKFZp686L193, DM | | | | |
| | 68D, MGC142218, PI3-K- | | | | |
| | C2(ALPHA), PI3-K-C2A, PI3K-C2 | phosphoinositide-3-kinase, class | | | |
| PIK3C2A | ALPHA, Pik3c2a_predicted | 2, alpha polypeptide | | 5286 | |
| | Ab1-346, A1649309, Angiostatin, | | | | |
| | DKFZp779M0222, GLU | | | | |
| | PLASMINOGEN, GLU-PG, Pg, | | | | |
| | PG2, Plasmin, PLASMIN HUMAN | | | | |
| PLG | PROTEIN, PLASMINOGEN, Scdp | plasminogen | | 5340 | |
| | A230024N07Rik, AA755424, | | | | |
| | AI385634, AI843499, Hox, K-2, | | | | |
| PRRX1 | Mhox, PHOX1, Pmx, PMX1, PRX1 | paired related homeobox 1 | | 5396 | |
| | alpha-type, C2, HC2, Macropain | | | | |
| | subunit C2, MGC14542, | | | | |
| | MGC14575, MGC14751, | | | | |
| | MGC1667, MGC21459, | | | | |
| | MGC22853, MGC23915, NU, | | | | |
| | PMSA1, PROS30, PROTEASOME | proteasome (prosome, | | | |
| | COMPONENT C2, Proteasome nu | macropain) subunit, alpha type, | | | |
| PSMA1 | chain | 1 | IPI00016832 | 5682 | 2.0 |
| | HC3, Lmpc3, Macropain subunit | | | | |
| | C3, MU, PMSA2, PROTEASOME | | | | |
| | C3 SUBUNIT, PROTEASOME | proteasome (prosome, | | | |
| | COMPONENT C3, PROTEASOME | macropain) subunit, alpha type, | | | |
| PSMA2 | SUBUNIT ALPHA TYPE2, PSC2 | 2 | | 5683 | |
| | AU045357, AW108089, BETA 2 | | | | |
| | PROTEASOME SUBUNIT, C7-I, | | | | |
| | D4Wsu33e, HC7-I, MGC104215, | proteasome (prosome, | | | |
| PSMB2 | MGC126885 | macropain) subunit, beta type, 2 | | 5690 | |
| | AL033320, C10, C10-II, HC10-II, | | | | |
| | MGC106850, MGC4147, | proteasome (prosome, | | | |
| PSMB3 | Proteasome chain 13, RC10-II | macropain) subunit, beta type, 3 | | 5691 | |
| | 26s proteasome subunit 4 ATPase, | | | | |
| | CIP21, MGC103150, MGC13687, | | | | |
| | MGC23214, MGC8570, MIP224, | | | | |
| | PROTEASOMAL ATPASE TAT- | proteasome (prosome, | | | |
| | BINDING PROTEIN 7, RPT3, S6, | macropain) 26S subunit, | | | |
| PSMC4 | S6B, TBP7 | ATPase, 4 | | 5704 | |
| | LOC81827, mSUG1, p45, p45 | proteasome (prosome, | | | |
| | Atpase, p45/SUG, S8, SUG1, | macropain) 26S subunit, | | | |
| PSMC5 | TBP10, TRIP1 | ATPase, 5 | | 5705 | |
| | | proteasome (prosome, | | | |
| | AW554874, dJ889N15.2, | macropain) 26S subunit, non- | | | |
| PSMD10 | GANKYRIN, p28 | ATPase, 10 | | 5716 | |
| | | proteasome (prosome, | | | |
| | 1500002F15Rik, AI480719, | macropain) 26S subunit, non- | | | |
| PSMD12 | MGC75406, MGC94696, P55 | ATPase, 12 | | 5718 | |
| | 1500032A03Rik, AW475925, | | | | |
| | KIAA0072, MGC23145, | proteasome (prosome, | | | |
| | mKIAA0072, Psmd5_predicted, | macropain) 26S subunit, non- | | | |
| PSMD5 | S5B, W91691 | ATPase, 5 | | 5711 | |
| | | proteasome (prosome, | | | |
| | AW107203, MOV34, MOV34L, | macropain) 26S subunit, non- | | | |
| PSMD7 | P40, Psmd7_predicted, S12 | ATPase, 7 (Mov34 homolog) | | 5713 | |
| | 6720456J22Rik, AA407360, | | | | |
| | AL033291, AL033322, AL033323, | proteasome (prosome, | | | |
| | C76433, HIP6, HYPF, MGC1660, | macropain) 26S subunit, non- | | | |
| PSMD8 | Nin1p, p31, S14 | ATPase, 8 | | 5714 | |
| PSMD9 | 1500011J20Rik, 2610202L11Rik, | proteasome (prosome, | IPI00010860 | 5715 | 2.0 |
| | Bridge, Bridge-1, MGC8644, P27 | macropain) 26S subunit, non- | | | |
| | | ATPase, 9 | | | |
| | AW413925, IFI5111, MGC113815, | | | | |
| | MGC8628, PA28A, PA28ALPHA, | proteasome (prosome, | | | |
| | Proteasome (prosome, macropain) | macropain) activator subunit 1 | | | |
| PSME1 | 28 subunit, alpha, REGalpha | (PA28 alpha) | | 5720 | |
| | AA589371, AI788882, PA28B, | | | | |
| | PA28b-p, PA28b2, PA28BETA, | proteasome (prosome, | | | |
| | Psme2-like, Psme2b, Psme2b-ps, | macropain) activator subunit 2 | | | |
| PSME2 | REGbeta | (PA28 beta) | IPI00384051 | 5721 | 2.0 |
| | DKFZP564B163, Gtbp, | | | | |
| | mKIAA3012, RAB1, Rab1p, Rab1r, | RAB1A, member RAS | | | |
| RAB1A | Ypt1 | oncogene family | | 5861 | |
| | 2610028L11Rik, AA419671, RAB6, | RAB6A, member RAS | | | |
| RAB6A | RAB6A', RAB6B | oncogene family | | 5870 | |
| | AL023026, D5Ertd559e, | ras-related C3 botulinum toxin | | | |
| | MGC111543, MIG5, p21-RAC, p21- | substrate 1 (rho family, small | | | |
| RAC1 | Rac1, Rac, TC-25 | GTP binding protein Rac1) | IPI00010271 | 5879 | 2.0 |
| | OSRC, p105, p110 RB, PRB, RB, | | | | |
| | RB-ASSOCIATED PROTEIN, | retinoblastoma 1 (including | | | |
| RB1 | RETINOBLASTOMA | osteosarcoma) | | 5925 | |
| | Cbp50, FLJ37041, PIG20, RCAL, | reticulocalbin 1, EF-hand | | | |
| RCN1 | RCN, Rcn1_predicted | calcium binding domain | IPI00015842 | 5954 | 2.0 |
| | | v-rel reticuloendotheliosis viral | | | |
| | | oncogene homolog A, nuclear | | | |
| | MGC131774, NF-kappaB, NFkB, | factor of kappa light polypeptide | | | |
| | NFKB P65, NFKB/P65, NFKB3, | gene enhancer in B-cells 3, p65 | | | |
| RELA | P65, p65 Nfkb, p65RelA | (avian) | | 5970 | |
| | | REX4, RNA exonuclease 4 | | | |
| REXO4 | Gm111, XPMC2, XPMC2H | homolog (S. cerevisiae) | | 57109 | |
| | 40S ribosomal protein S5, | | | | |
| RPS5 | AA617411 | ribosomal protein S5 | IPI00008433 | 6193 | 2.0 |
| | 60B8AG, AI323541, B8Ag, CAGA, | | | | |
| | calgranulin A, CALPROTECTIN, | | | | |
| | CFAG, CGLA, CP-10, CYSTIC | | | | |
| | FIBROSIS ANTIGEN, L1Ag, | | | | |
| | MA387, MIF, MIGRATION | | | | |
| | INHIBITORY FACTOR RELATED | | | | |
| S100A8 | PROTEIN 8, MRP8, NIF, P8 | S100 calcium binding protein A8 | IPI00007047 | 6279 | 2.0 |
| | AA959608, AW108331, | | | | |
| | MGC22217, RATRYUDOCA, | | | | |
| | RYUDOCA, RYUDOCAN, SYND4, | | | | |
| SDC4 | SYNDECAN-4 | syndecan 4 | | 6385 | |
| | | sema domain, immunoglobulin | | | |
| | | domain (Ig), transmembrane | | | |
| | CD100, coll-4, M-sema-G, Sema H, | domain (TM) and short | | | |
| | Sema4d_predicted, Semacl2, | cytoplasmic domain, | | | |
| SEMA4D | SEMAJ, Semcl2 | (semaphorin) 4D | | 10507 | |
| | 1110036M19RIK, AI462524, | | | | |
| | AI646751, MASPIN, ovalbumin, | serpin peptidase inhibitor, clade | | | |
| SERPINB5 | PI5, Spi7 | B (ovalbumin), member 5 | | 5268 | |
| | AI114908, ANTITHROMBIN, | | | | |
| | ANTITHROMBIN III, | | | | |
| | ANTITHROMBIN3, AT, AT3, ATIII, | serpin peptidase inhibitor, clade | | | |
| SERPINC1 | MGC22579 | C (antithrombin), member 1 | IPI00165421 | 462 | 2.0 |
| | | serpin peptidase inhibitor, clade | | | |
| | AI195227, Dmrs91, EPC-1, PEDF, | F (alpha-2 antiplasmin, pigment | | | |
| | PEDFL, PIG35, pigment epithelium- | epithelium derived factor), | | | |
| SERPINF1 | derived factor, Sdf3 | member 1 | IPI00006114 | 5176 | 2.0 |
| | | surfactant, pulmonary- | | | |
| SFTPC | PSP-C, SFTP2, SP-C, SP5 | associated protein C | | 6440 | |
| | AA409932, AI314629, AU015566, | | | | |
| | BIF1, CGI-61, dJ612B15.2, | | | | |
| | endophilin B1, KIAA0491, | SH3-domain GRB2-like | | | |
| SH3GLB1 | mKIAA0491 | endophilin B1 | | 51100 | |
| | 1200007H22Rik, 1700125L08Rik, | | | | |
| | 5830464D22Rik, AI447724, CIN85, | SH3-domain kinase binding | | | |
| SH3KBP1 | GIG10, HSB-1, MIG18, RUK, SETA | protein 1 | | 30011 | |
| | MGC10182, SLUG, Slugh, | | | | |
| SNAI2 | SLUGH1, Snail2, WS2D | snail homolog 2 (Drosophila) | | 6591 | |
| | 23kDa, AA408749, HsT17016, | synaptosomal-associated | | | |
| SNAP23 | SNAP23A, SNAP23B, Sndt, Syndet | protein, 23kDa | | 8773 | |
| | AD AMYLOID, ALPHA | | | | |
| | SYNUCLEIN, ALPHASYN, | | | | |
| | MGC105443, MGC110988, NACP, | synuclein, alpha (non A4 | | | |
| | PARK1, PARK4, PD1, SYNUCLEIN | component of amyloid | | | |
| SNCA | ALPHA | precursor) | IPI00024107 | 6622 | 2.0 |
| | 2410133M08RIK, dJ423B22.2, | Smad nuclear interacting protein | | | |
| SNIP1 | FLJ12553, RP3-423B22.3 | 1 | IPI00154515 | 79753 | 2.0 |
| | 2310008B08Rik, AW048543, Bx42, | | | | |
| | MGC119379, NCOA-62, Prp45, | | | | |
| | PRPF45, RGD1561926_predicted, | | | | |
| SNW1 | SKIIP, SKIP, SNW1 protein | SNW domain containing 1 | | 22938 | |
| | 1110003E12RIK, AA450830, | | | | |
| | A1845540, Sp1 (trans spliced | | | | |
| SP1 | isoform), SP1-1 | Sp1 transcription factor | | 6667 | |
| | AA960535, AI790405, Apl-1, | | | | |
| | BNSP, Bone Sialoprotein, Bopn, | | | | |
| | Bsp, BSPI, Eta, ETA-1, | | | | |
| | MGC110940, Minopontin, | | | | |
| | NEPHROPONTIN, OP, OPN, Opnl, | secreted phosphoprotein 1 | | | |
| | OSP, OSTEOPONTIN, Ric, | (osteopontin, bone sialoprotein | | | |
| | Secreted phosphoprotein 1, | I, early T-lymphocyte activation | | | |
| SPP1 | UROPONTIN | 1) | IPI00021000 | 6696 | 2.0 |
| | AF093576, AI451697, ALPHA1 | | | | |
| | SPECTRIN, EL2, Erythroid Alpha- | | | | |
| | Spectrin, HA, Spectrin alpha 1, | spectrin, alpha, erythrocytic 1 | | | |
| SPTA1 | Sph, Spna-1, Spna1, SPTA | (elliptocytosis 2) | IPI00220741 | 6708 | 2.0 |
| | (ALPHA)II-SPECTRIN, | | | | |
| | 2610027H02Rik, A2a, Alpha fodrin, | | | | |
| | ALPHA SPECTRIN, ALPHA | | | | |
| | SPECTRIN 2, Alpha-spectorin, | | | | |
| | Alphall spectrin, FLJ44613, IPF, | spectrin, alpha, non-erythrocytic | | | |
| SPTAN1 | Spectrin alpha 2, SPNA2 | 1 (alpha-fodrin) | IPI00413728 | 6709 | 2.0 |
| | AI842465, Beta1 Spectrin, | | | | |
| | D330027P03, D330027P03Rik, | | | | |
| | ERYTHROCYTIC BETA | | | | |
| | SPECTRIN, Gm1301, HSpTB1, Ja, | | | | |
| | jaundiced, KIAA4219, LOC314251, | spectrin, beta, erythrocytic | | | |
| | mKIAA4219, RGD:1303243, | (includes spherocytosis, clinical | | | |
| SPTB | SPNB1 | type I) | IPI00216513 | 6710 | 2.0 |
| | LA, LA ANTIGEN, La protein, | | | | |
| | LARP3, Lupus la protein homolog, | Sjogren syndrome antigen B | | | |
| SSB | MGC118101, MGC93380, SSB-48 | (autoantigen La) | IPI00009032 | 6741 | 2.0 |
| | | stress-induced-phosphoprotein | | | |
| | HOP, IEF-SSP-3521, mSTI1, P60, | 1 (Hsp70/Hsp90-organizing | | | |
| STIP1 | STI1, STI1L | protein) | IPI00013894 | 10963 | 2.0 |
| | COFACTOR A, Tcp1-chaperonin | | | | |
| TBCA | cofactor a | tubulin folding cofactor A | IPI00217236 | 6902 | 2.0 |
| | BETA IG-H3, BIGH3, CED, DPD1, | transforming growth factor, beta | | | |
| TGFB1 | TGF-BETA, TGF-BETA1, TGFB | 1 | | 7040 | |
| THBS2 | THROMBOSPONDIN 2, TSP-2 | thrombospondin 2 | IPI00018769 | 7058 | 2.0 |
| | AI528729, C130033P17Rik, HXB, | | | | |
| | MGC144208, MGC144209, TEN, | | | | |
| TNC | TENASCIN C, TN | tenascin C (hexabrachion) | IPI00031008 | 3371 | 2.0 |
| | AT-TNF, DIF, Differentiation- | | | | |
| | induced Factor, MGC124630, | | | | |
| | MGC151434, RATTNF, TMTNF, | | | | |
| | TNF-ALPHA, TNFA, Tnfsf1a, | tumor necrosis factor (TNF | | | |
| TNF | TNFSF2 | superfamily, member 2) | | 7124 | |
| | | tumor necrosis factor, alpha- | | | |
| TNFAIP6 | TNFIP6, TSG-6 | induced protein 6 | | 7130 | |
| | MGC29565, MGC93568, OCIF, | tumor necrosis factor receptor | | | |
| | OPG, OSTEOPROTEGERIN, | superfamily, member 11b | | | |
| TNFRSF11B | TNFR11, TR1 | (osteoprotegerin) | | 4982 | |
| | bbl, bfy, bhy, Delta N p53, LFS1, | tumor protein p53 (Li-Fraumeni | | | |
| TP53 | MGC112612, P53, TRP53 | syndrome) | | 7157 | |
| | 2410002K23Rik, HSP75, HSP90L, | TNF receptor-associated protein | | | |
| TRAP1 | MGC95278 | 1 | IPI00030275 | 10131 | -2.0 |
| | HTRP-1, MGC133334, | | | | |
| | MGC133335, Mtrp1, Transient | transient receptor potential | | | |
| | receptor protein 1, TRP1, TRP1 | cation channel, subfamily C, | | | |
| TRPC1 | ALPHA, Trrp1 | member 1 | | 7220 | |
| | GRIM-12, KM 102 DERIVED | | | | |
| | REDUCTASE LIKE FACTOR, | | | | |
| | MGC9145, MGC93353, Tgr, | | | | |
| | Thioredoxin reductase, TR, TR1, | | | | |
| TXNRD1 | TRXR1, TXNR | thioredoxin reductase 1 | IPI00472175 | 7296 | 2.0 |
| | AW822034, C88048, Gad, PARK5, | ubiquitin carboxyl-terminal | | | |
| | Pgp, PGP9.5, Protein gene product | esterase L1 (ubiquitin | | | |
| UCHL1 | 9.5, R75593, UCH | thiolesterase) | IPI00018352 | 7345 | 2.0 |
| | | vasodilator-stimulated | | | |
| VASP | AA107290, Vasp_predicted | phosphoprotein | | 7408 | |
| | 1700102N10RIK, BP-8, Byb1, | | | | |
| | C79409, Cbfa, CSDA2, CSDB, | | | | |
| | DBPB, DBPB-LIKE, EF1A, MDR- | | | | |
| | NF1, MGC104858, MGC110976, | | | | |
| | MGC117250, MGC118070, | | | | |
| | MGC127824, MSY1, mYB-1a, | | | | |
| | NSEP-1, THYROID Y BOX | | | | |
| | PROTEIN, VACSSBF1, Y-box | | | | |
| | Binding Protein1, YB-1, YBP1, | | | | |
| YBX1 | Ybx1-ps3, YEBP | Y box binding protein 1 | | 4904 | |
| | 14-3-3, 14-3-3 protein theta, 14-3-3 | | | | |
| | TAU, 14-3-3 THETA, 14-3-3t, 1C5, | tyrosine 3- | | | |
| | 2700028P07Rik, AA409740, | monooxygenase/tryptophan 5- | | | |
| | AU021156, HS1, MGC118161, | monooxygenase activation | | | |
| YWHAQ | R74690, RP23-402H11.1 | protein, theta polypeptide | IPI00018146 | 10971 | -2.0 |

All proteins from Table II or Table III would be suitable to be a plaque marker for future cardiovascular events. Due to practical reasons, however, a reduction of the list was applied. For this, we choose to select for proteins that showed a cardiovascular phenotype in the mouse using the Mouse Genome Informatics (MGI) database (Mouse Genome Informatics Web Site, The Jackson Laboratory, Bar Harbor, Maine. World Wide Web (URL: http://www.informatics.jax.org). Since not every protein has a described phenotype and since the existence of a cardiovascular phenotype was not investigated for every protein, this selection was for practical reasons only. It is believed that all the proteins listed in Table I and/or Table II and/or Table III would be suitable as a marker according to the invention

The 16 proteins that have a cardiovascular phenotype are listed in table IV.

**Table IV List of 16 human plaque proteins that differ between patients that had a major cardiovascular events within 2 years after the removal of the carotid plaque (carotid atherectomy) and sex and age matched controls and that were selected by Ingenuity for building networks and were present in 1 major network and had a cardiovascular phenotype in the mouse.**

| International Protein Index | Name | Description |
|---|---|---|
| IPI00218446 | ADH5 | alcohol dehydrogenase 5 (class III), chi polypeptide |
| IPI00018342 | AK1 | adenylate kinase 1 |
| IPI00418169 | ANXA2 | annexin A2 |
| IPI00002459 | ANXA6 | annexin A6 |
| IPI00215746 | FABP4 | fatty acid binding protein 4, adipocyte |
| IPI00028888 | HNRPD | heterogeneous nuclear ribonucleoprotein D (AU-rich element RNA binding protein 1, 37kDa) |
| | | integrin, beta 1 (fibronectin receptor, beta polypeptide, antigen CD29 includes MDF2, |
| IPI00217561 | ITGB1 | MSK12) |
| IPI00021405 | LMNA | lamin A/C |
| IPI00010271 | RAC1 | ras-related C3 botulinum toxin substrate 1 (rho family, small GTP binding protein Rac1) |
| IPI00165421 | SERPINC1 | serpin peptidase inhibitor, clade C (antithrombin), member 1 |
| | | serpin peptidase inhibitor, clade F (alpha-2 antiplasmin, pigment epithelium derived factor), |
| IPI00006114 | SERPINF1 | member 1 |
| IPI00021000 | SPP1 | secreted phosphoprotein 1 (osteopontin, bone sialoprotein I, early T-lymphocyte activation 1) |
| IPI00216513 | SPTB | spectrin, beta, erythrocytic (includes spherocytosis, clinical type I) |
| IPI00018769 | THBS2 | thrombospondin 2 |
| IPI00472175 | TXNRD1 | thioredoxin reductase 1 |
| IPI00018352 | UCHL1 | ubiquitin carboxyl-terminal esterase L1 (ubiquitin thiolesterase) |

One of these proteins was initially selected for the individual verification and proof of concept of the potency of these lists. The best candidate would be a protein from the network with the highest score (network 1). Next, we investigated the Online Mendelian Inheritance in Man (OMIM ™) database (McKusick-Nathans Institute of Genetic Medicine, Johns Hopkins University (Baltimore, MD) and National Center for Biotechnology Information, National Library of Medicine (Bethesda, MD). World Wide Web URL: http://www.ncbi.nlm.nih.gov/omiml) for the proteins from this network and the role of these proteins in processes playing a potential role in plaque rupture: inflammation, collagen turn-over and coagulation. Further, selection was dependent on the availability of a commercial ELISA for a protein. As a result, SPP1 (IPI00021000, Osteopontin) was selected for validation on the individual patients of the AtheroExpress database.

## Claims

1. A biomarker in atherosclerotic plaque associated with an increased risk of suffering a cardiovascular event, wherein said biomarker comprises at least one protein that is a member of at least one Ingenuity® network selected from the group consisting of:
a) the Ingenuity® network defined by the proteins ACTR2, CAPN2, CAST, FSCN1, HCLS1, HNRPA2B1, HNRPC, HSP90AA1, IGFBP7, MYLK, RAC1, S100A8, SERPINF1, SNCA, SPP1, SSB, STIP1, UCHL1, and YWHAQ;
b) the Ingenuity® network defined by the proteins AHNAK, AKR1A1, C1QB, C1QC, CBR3, CFD, CORO1C, FBP1, HNRPC, HNRPD, LAMP1, NEDD8, PSME2, SERPINC1, SPTA1, and TNC;
c) the Ingenuity® network defined by the proteins C19ORF10, CAPZA1 (includes EG:829), CAPZA2, CAPZB, DDX23, EEF1A2, FSCN1, GOLGA4, HSPA5, KRT1, M6PRBP1, NPC2, PGD, S100A8, SNIP1, and SPTAN1;
d) the Ingenuity® network defined by the proteins ADH5, AK1, CALU (includes EG:813), CRYZ, DPYSL3, EIF3S3, HNRPA2B1, IGFALS, IGHG1, MRLC2, NP, PDLIM2, and TXNRD1;
e) the Ingenuity® network defined by the proteins AGC1, ANXA2, C6, CLTC, ERAF, FABP4, ITGB1, LMNA, RPS5, SPTB, THBS2, and TNC;
f) the Ingenuity® network defined by the proteins CLEC3B, HAPLN1, HNRPD, MDH2, PCOLCE, PSMA1, PSMD9, RCN1, SERPINC1, TBCA, TNC, and TRAP1;
g) the Ingenuity® network defined by protein RNPEP, and
h) the Ingenuity® network defined by the protein TWF2.

2. The biomarker of claim 1, wherein said biomarker is a member of the single Ingenuity® network composed of the networks as defined under a)-f) of which the proteins are listed in table III.

3. The biomarker of claim 1 or 2, wherein said biomarker comprises at least one protein selected from the group consisting of ACTR2, ADH5, AGC1, AHNAK, AK1, AKR1A1, ANXA2, ANXA6, C19ORF10, C1QB, C1QC, C6, CALU (includes EG:813), CAPN2, CAPZA1 (includes EG:829), CAPZA2, CAPZB, CAST, CBR3, CDC37, CFD, CLEC3B, CLTC, CORO1C, CRYZ, DDAH2, DDX23, DPYSL3, EEF1A2, EFHD1, EIF3S3, ERAF, FABP4, FBP1, FSCN1, GOLGA4, HAPLN1, HCLS1, HNRPA2B1, HNRPC, HNRPD, HSP90AA1, HSPA5, IGFALS, IGFBP7, IGHG1, ITGB1, KRT1, LAMP1, LMNA, M6PRBP1, MDH2, MRLC2, MYLK, NEDD8, NP, NPC2, PCOLCE, PDLIM2, PGD, PSMA1, PSMD9, PSME2, RAC1, RCN1, RNPEP, RPS5, S100A8, SERPINA6, SERPINC1, SERPINF1, SNCA, SNIP1, SPP1, SPTA1, SPTAN1, SPTB, SSB, STIP1, TBCA, THBS2, TNC, TRAP1, TWF2, TXNRD1, UCHL1, and YWHAQ.

4. The biomarker of claim 1 or 2, wherein said biomarker comprises at least one protein selected from the group consisting of ACTR2, ADH5, AGC1, AHNAK, AK1, AKR1A1, ANXA2, ANXA6, C19ORF10, C1QB, C1QC, C6, CALU (includes EG:813), CAPN2, CAPZA1 (includes EG:829), CAPZA2, CAPZB, CAST, CBR3, CDC37, CFD, CLEC3B, CLTC, CORO1C, CRYZ, DDX23, DPYSL3, EEF1A2, EIF3S3, ERAF, FABP4, FBP1, FSCN1, GOLGA4, HAPLN1, HCLS1, HNRPA2B1, HNRPC, HNRPD, HSP90AA1, HSPA5, IGFALS, IGFBP7, IGHG1, ITGB1, KRT1, LAMP1, LMNA, M6PRBP1, MDH2, MRLC2, MYLK, NEDD8, NP, NPC2, PCOLCE, PDLIM2, PGD, PSMA1, PSMD9, PSME2, RAC1, RCN1, RPS5, S100A8, SERPINC1, SERPINF1, SNCA, SNIP1, SPP1, SPTA1, SPTAN1, SPTB, SSB, STIP1, TBCA, THBS2, TNC, TRAP1, TXNRD1, UCHL1, and YWHAQ.

5. The biomarker of claim 1 or 2, wherein said biomarker comprises at least one protein selected from the group consisting of ADH5, AK1, ANXA2, ANXA6, FABP4, HNRPD, ITGB1, LMNA, RAC1, SERPINC1, SERPINF1, SPP1, SPTB, THBS2, TXNRD1, and UCHL1.

6. The biomarker of claim 1 or 2, wherein said biomarker is SPP1 (osteopontin).

7. A method for predicting the risk of a subject developing a medical condition comprising detecting a biomarker according to any one of claims 1-6 in (a sample of) atherosclerotic plaque from said subject.

8. The method of claim 7, wherein said atherosclerotic plaque is atherosclerotic plaque in a coronary, femoral and/or carotid artery.

9. The method of claim 7 or 8, wherein said medical condition is a cardiovascular event.

10. The method of claim 9, wherein said cardiovascular event is vascular death or sudden death, non fatal stroke, non fatal myocardial infarction, non fatal rupture of an abdominal aortic aneurysm, rupture of abdominal aortic aneurysm confirmed by laparatomy, vascular intervention or restenosis.

11. A method for predicting the risk of a test subject developing a medical condition comprising the steps of:
a) typing atherosclerotic plaque, comprising the steps of:
- measuring the amount of at least one protein in a first (sample of) atherosclerotic plaque of a reference subject at risk of developing a medical condition and in a second (sample of) atherosclerotic plaque of a control subject not at risk of developing said medical condition, or providing a protein profile for said first and second (sample of) atherosclerotic plaque,
- determining whether said at least one protein is differentially present in said first (sample of) atherosclerotic plaque compared to said second (sample of) atherosclerotic plaque, or determining the differential protein expression profile between said first and second (sample of) atherosclerotic plaque,
- correlating the risk of developing said medical condition to said differentially present protein or to said differential protein expression profile,
- identifying said differentially present protein or said differential protein expression profile as a biomarker in case said correlation is statistically significant,
and
b) detecting said biomarker in (a sample of) atherosclerotic plaque from said test subject, wherein said test subject is at risk of developing said medical condition when said biomarker is present in said (sample of) atherosclerotic plaque.

12. The method of claim 11, wherein said medical condition is a cardiovascular event.

13. The method of any one of claims 7-12, wherein said step of:
- measuring the amount of at least one protein;
- providing a protein profile, and/or
- detecting said biomarker,
is performed by using mass spectrometry, protein chip array analysis, antibody array analysis, immunoassay analysis, MRI, NMR, ultrasound spectroscopy, Raman spectroscopy and/or infra red spectroscopy.

14. Kit of parts for performing a method according to any one of claims 6-12, comprising at least one biomarker as defined in any one of claims 1-5, or an antibody that binds specifically to said biomarker, said kit of parts optionally further comprising one or more of the following:
- at least one reference or control sample;
- information on the reference value for the biomarker;
- at least one peptide capable of binding to said antibody;
- at least one detectable marker for detecting binding between said biomarker and said antibody.

15. Antibody microarray for performing a method according to any one of claims 7-13, comprising antibodies that bind specifically to at least two biomarkers as defined in any one of claims 1-6 bound to a solid support.

16. A diagnostic reagent capable of specifically binding to a biomarker as defined in any of claims 1-6.

17. The diagnostic reagent of claim 16, wherein said diagnostic reagent is an antibody or a derivative thereof.

18. A diagnostic composition comprising a diagnostic reagent according to claim 16 or 17.

19. Use of a biomarker as defined in any one of claims 1-6 for predicting the risk of a cardiovascular event in a subject.

20. Use of a diagnostic composition according to claim 18, for diagnosing the risk of a cardiovascular event in a subject.

21. A method of treating a subject having an increased risk of suffering a cardiovascular event, said method comprising using a biomarker as defined in any one of claims 1-6 as a therapeutic target or as a therapeutic agent.

22. The method of claim 21, wherein said use of said biomarker as a therapeutic target or as a therapeutic agent comprises affecting the expression of at least one protein that is a member of at least one Ingenuity® network selected from the group consisting of:
a) the Ingenuity® network defined by the proteins ACTR2, CAPN2, CAST, FSCN1, HCLS1, HNRPA2B1, HNRPC, HSP90AA1, IGFBP7, MYLK, RAC1, S100A8, SERPINF1, SNCA, SPP1, SSB, STIP1, UCHL1, and YWHAQ;
b) the Ingenuity® network defined by the proteins AHNAK, AKR1A1, C1QB, C1QC, CBR3, CFD, CORO1C, FBP1, HNRPC, HNRPD, LAMP1, NEDD8, PSME2, SERPINC1, SPTA1, and TNC;
c) the Ingenuity® network defined by the proteins C19ORF10, CAPZA1 (includes EG:829), CAPZA2, CAPZB, DDX23, EEF1A2, FSCN1, GOLGA4, HSPA5, KRT1, M6PRBP1, NPC2, PGD, S100A8, SNIP1, and SPAN1;
d) the Ingenuity® network defined by the proteins ADH5, AK1, CALU (includes EG:813), CRYZ, DPYSL3, EIF3S3, HNRPA2B1, IGFALS, IGHG1, MRLC2, NP, PDLIM2, and TXNRD1;
e) the Ingenuity® network defined by the proteins AGC1, ANXA2, C6, CLTC, ERAF, FABP4, ITGB1, LMNA, RPS5, SPTB, THBS2, and TNC;
f) the Ingenuity® network defined by the proteins CLEC3B, HAPLN1, HNRPD, MDH2, PCOLCE, PSMA1, PSMD9, RCN1, SERPINC1, TBCA, TNC, and TRAP1;
g) the Ingenuity® network defined by protein RNPEP, and
h) the Ingenuity® network defined by the protein TWF2.

23. The method of claim 21 or 22, wherein said use of said biomarker as a therapeutic target comprises decreasing the amount of at least one protein that is over-expressed in subjects having an increased risk of suffering a cardiovascular event, or increasing the amount of at least one protein that is under-expressed in subjects having an increased risk of suffering a cardiovascular event.

24. The method of claim 23, wherein said protein that is over-expressed is SPP1 (osteopontin).

25. Pharmaceutical composition for the treatment of an increased risk of suffering a cardiovascular event, comprising at least one inhibitor compound selected from:
- an antibody or derivative thereof directed against the biomarker of any one of claims 1-6, preferably a biomarker expressed on the cell membrane, and said derivative preferably being selected from the group consisting of scFv fragments, Fab fragments, chimeric antibodies, bifunctional antibodies, intrabodies, and other antibody-derived molecules;
- a biomarker of any one of claims 1-6
- a small molecule interfering with the biological activity of said biomarker.
- an antisense molecule, in particular an antisense RNA or antisense oligodeoxynucleotide.
- an RNAi molecule and
- a ribozyme,
and a suitable excipient, carrier or diluent.

26. Method of treating a subject, comprising administering to said subject the pharmaceutical composition of claim 25 in an amount effective to decrease or prevent the risk of said subject suffering a cardiovascular event.
